# EUROPEAN PATENT SPECIFICATION

(11) **EP 4 615 274 B1**
(45) Date of publication and mention of the grant of the patent: **01.04.2026**
(21) Application number: 23800440.2
(22) Date of filing: 01.11.2023
(51) Int. Cl.: A46B 15/00

(54) **ORAL CARE SYSTEM**
MUNDPFLEGESYSTEM
SYSTÈME D'HYGIÈNE BUCCALE

(30) Priority: 07.11.2022 EP 22205831
(43) Date of publication of application: 17.09.2025
(73) Proprietor: Koninklijke Philips N.V., 5656 AG Eindhoven (NL)
(72) Inventor: GERHARDT, Lutz Christian, 5656 AG Eindhoven (NL); PANDE, Nakul, 5656 AG Eindhoven (NL); HIWALE, Sujitkumar, 5656 AG Eindhoven (NL); KOOIJMAN, Gerben, 5656 AG Eindhoven (NL)
(74) Representative: Philips Intellectual Property & Standards
(86) International application number: PCT/EP2023/080453
(87) International publication number: WO 2024/099844

(56) References cited:
- US-A1- 2015 297 085
- US-B2- 10 179 038

## Description

### FIELD OF THE INVENTION

This invention relates to an oral care system.

### BACKGROUND OF THE INVENTION

Build-up of calcified deposits in the body can be problematic. An example of a calcified deposit is dental tartar.

Dental tartar, or early dental calculus, is a risk factor for gingivitis and periodontitis. Dental tartar has different calcium phosphate crystal phases which differ in solubility. The poor aqueous solubility of some of the calcium phosphate crystal phases can make the dental tartar difficult to remove.

At present, dental tartar tends to be treated/removed only by dental practitioners and oral hygienists using mechanical or ultrasound scalers.

Such removal of dental tartar tends to be relatively safe, as it is implemented by an oral healthcare professional. However, in-office procedures require the patient/subject to travel to visit the dental office. Furthermore, mechanical- or ultrasound-assisted scraping to remove tartar tends to cause discomfort, and occasionally even pain, during the procedure.

It would be desirable to provide dental tartar-related treatment solutions that address such challenges, and particularly enable treatment to be safely and/or efficiently provided in a subject's own home.

US 2015/297085 A1 discloses a dental apparatus and method of use of the dental apparatus utilizing a low intensity excitation light having a first frequency or intensity to detect when the dental apparatus is placed in a mouth of a user and a high intensity excitation light which may have a second frequency to detect dental plaque, or treat a dental condition.

US 10179038B2 discloses improved oral care implements that incorporate one or more electrode pairs. Within each electrode pair, a sacrificial metal is used that breaks down when a potential difference is applied across the electrode pair. Ions are released when the metal breaks down, and those ions aid in oral health.

### SUMMARY OF THE INVENTION

The invention is defined by independent claim 1. The dependent claims define advantageous embodiments.

According to the invention there is provided an oral care system comprising: a detection system for detecting dental tartar within a subject's oral cavity; an electrochemical system for generating ions for delivery within the subject's oral cavity during an electrochemical procedure; and one or more processors configured to: obtain, via the detection system, a dental tartar indication indicative of dental tartar within the subject's oral cavity; and control the electrochemical procedure provided by the electrochemical system based on the dental tartar indication.

The term "dental tartar" as used herein may refer to an early phase of calculus comprising inorganic material and some organic material.

In some embodiments, the dental tartar comprises, e.g. is defined by, octacalcium phosphate-comprising calculus.

Electrochemically generated ions may be suitable for treating various dental tartar-related issues.

In some embodiments, the ions generated by the electrochemical system are one or more selected from hydrogen ions, zinc ions, tin ions, copper ions, and silver ions.

Hydrogen ions may assist to lower a pH in the oral cavity proximal to dental pieces, e.g. teeth. Such lowering of the pH may assist in dissolution of dental tartar.

In embodiments in which hydrogen ions are generated by the electrochemical system, the electrochemical system may be arranged to generate the hydrogen ions via water, e.g. water included in saliva, electrolysis.

Zinc ions, tin ions, copper ions, and/or silver ions may provide various oral health benefits, including, for instance, antimicrobial action.

In embodiments in which zinc ions, tin ions, copper ions, and/or silver ions are generated by the electrochemical system, the electrochemical system may include one or more zinc-comprising, tin-comprising, copper-comprising, and/or silver-comprising sacrificial electrodes.

More generally, the present disclosure is at least partly based on the insight that the presence, or otherwise, of dental tartar in the subject's oral cavity can beneficially be used as a guide for controlling the electrochemical system's generation of ions.

In particular, when the electrochemical system is configured to generate hydrogen ions, detection of dental tartar can justify lowering the pH proximal to the subject's dental pieces in order to dissolve the dental tartar. On the other hand, should no or only minimal quantities of dental tartar be detectable, it may instead be desirable to control the electrochemical system so as to avoid lowering this pH, or even to increase the pH following a previous delivery of hydrogen ions, to minimize the risk of dissolution of enamel. This is because the enamel, in common with the dental tartar, may be more easily dissolvable at lower pH conditions.

In embodiments in which the electrochemical system generates ions other than hydrogen ions, such as zinc ions, tin ions, copper ions, and/or silver ions, it may be desirable to restrict generation of such ions only to when dental tartar is detectable in the subject's oral cavity because of the dental tartar detection being indicative that therapeutic, e.g. antimicrobial, ion treatment is justified. On the other hand, should no or only minimal quantities of dental tartar be detectable, it may instead be desirable to control the electrochemical so as to avoid generation of such ions, for example to prolong the operating lifetime of the sacrificial electrodes from which the ions may be electrochemically generated.

The oral care system correspondingly comprises the detection system for detecting dental tartar within the subject's oral cavity, and one or more processors. The processor(s) is or are configured to obtain, via the detection system, a dental tartar indication within the subject's oral cavity, and control the electrochemical system to provide the electrochemical procedure, e.g. to electrochemically generate the ions and/or deliver the electrochemically generated ions within the subject's oral cavity, based on the dental tartar indication.

Dental tartar detection can, in particular, make lower pH-driven dental tartar dissolution safer to implement by assisting to lower the risk of uncontrolled and unwanted dissolution of healthy tooth-protecting enamel. This can mean that the oral care system according to the present disclosure may be used for at-home tartar removal.

The detection system may utilize any suitable dental tartar detection principle, for instance a detection principle that distinguishes one or more characteristics of dental tartar with respect to enamel.

For example, the detection system may include an ion detection system configured to detect dental tartar, e.g. via a release rate of one or more ions from enamel being different from, in particular lower than, a release rate of the one or more ions from dental tartar.

Such a release rate difference may reflect the fact that dental tartar is formed via transition from biofilm, which contains organic content, to calculus, which is mainly or even fully inorganic, via progressive calcification and crosslinked network-forming processes.

In some embodiments, the detection system may include a material hardness detection system configured to detect dental tartar via a hardness measurement, for example via a tapping element configured to tap against materials in the subject's oral cavity.

In such embodiments, the detection system may be configured to detect dental tartar based on a hardness difference between dental tartar and enamel.

Dental tartar tends to be softer that enamel, particularly in its early phases of formation.

In some embodiments, the detection system comprises an optical detection system for optically detecting dental tartar within the subject's oral cavity.

Optical detection of dental tartar has been found to be particularly advantageous at least partly owing to differences between dental tartar's and enamel's interaction with light. This may be due to the different chemical/structural properties of dental tartar as compared to tooth enamel.

The optical detection system may, for instance, comprise an imaging system for imaging the subject's oral cavity, and/or a non-imaging optical detection system, e.g. including photodiode(s) and optical fiber(s).

In some embodiments, the detection system comprises a spectral detection system configured to spectroscopically detect the presence of dental tartar within the subject's oral cavity. Thus, the dental tartar indication may be based on spectroscopic detection of the presence of dental tartar.

Such spectroscopic detection of the presence of dental tartar may capitalize on the different chemical/structural properties of dental tartar compared to enamel resulting in spectral differences between these materials that can be spectroscopically discerned. A key point of the present disclosure concerns use of spectral information to distinguish between dental tartar and enamel.

In some embodiments, the one or more processors is or are configured to execute a spectral comparison algorithm to distinguish between dental tartar and enamel, with the dental tartar indication comprising a result of the spectral comparison algorithm.

In other words, the spectral comparison algorithm, which can be alternatively termed a "spectral matching algorithm", may run on the processor(s) and the result of the spectral comparison algorithm may be used to generate control signals for controlling the electrochemical system. Such control signals may, for example, initiate or terminate the electrochemical procedure, e.g. (hydrogen) ion generation, based on the result of the spectral comparison algorithm.

In some embodiments, the spectroscopic detection of the presence of dental tartar within the subject's oral cavity comprises infrared spectroscopic detection. Infrared spectroscopic detection, e.g. using mid and/or near infrared radiation, may provide a relatively convenient and reliable way of distinguishing between enamel and dental tartar.

In such embodiments, the electrochemical system may be adaptively controlled, e.g. by switching the electrochemical treatment on or off, based on infrared optical measurement of material within the oral cavity.

When the electrochemical procedure comprises generating hydrogen ions for delivery within the subject's oral cavity, infrared optical measurement-based control may provide a particularly convenient and reliable way of assisting to minimize the risk of uncontrolled/unwanted dissolution of healthy enamel.

In some embodiments, the electrochemical system comprises a control arrangement and one or more pairs of conductive element such as electrodes for contacting an aqueous solution. In such embodiments, the one or more processors may be configured to, based on the dental tartar indication indicating the presence of dental tartar, trigger the control arrangement to provide an electrical signal that causes at least one electrode of the one or more pairs of electrodes to generate hydrogen ions from water in the aqueous solution to deliver within the subject's oral cavity.

In such embodiments, the hydrogen ions may assist to lower a local pH proximal to the subject's teeth to assist dissolution of dental tartar.

A variation of the control signal may control the generation of the hydrogen ions and/or neutralization thereof.

The variation, for example time-variation, of the electrical signal may provide control over the generation of hydrogen ions which contrasts with, for instance, an uncontrolled generation of hydrogen ions via a non-varying voltage across the pair of electrodes provided by a continuously applied direct current source. This may permit the local pH to which the subject's teeth are exposed to be more closely controlled, thereby facilitating at-home use of the oral care system.

It is noted that the term "electrical signal" as used herein may refer to a voltage or a current signal. Alternatively, an electrical impedance may be controlled.

In some embodiments, the variation of the electrical signal limits a rate of change of generation of the hydrogen ions, for example relative to a non-varying voltage, of equivalent magnitude to the electrical signal's peak amplitude, across the pair of electrodes provided by a direct current source.

Alternatively or additionally, the variation of the electrical signal may promote hydrogen ion neutralization, for example by the electrical signal comprising or being defined by a bidirectional waveform supplied to the electrodes. The neutralization promotion provided by such a bidirectional waveform will be explained in more detail herein below.

In some embodiments, the electrical signal comprises a periodic variation in at least one of amplitude, polarity and frequency. This may assist to control the build-up of hydrogen ions, and thus assist in controlling the local pH.

Alternatively or additionally, the electrical signal comprises a bidirectional waveform. Alternating the polarity of the electrodes via such a bidirectional waveform may assist to limit the progression of a low pH "front" emanating from the electrodes, and stabilize the pH. This is because, following a change in polarity, previously generated hydrogen ions at one of the electrodes may be neutralized by currently generated hydroxide ions at the same electrode.

In some embodiments, a positive peak amplitude of the bidirectional waveform is different from a negative peak amplitude of the bidirectional waveform. This may result in a continuous, but controlled increase or decrease in pH at a particular location.

In some embodiments, one of the positive and negative peak amplitudes is at least 1.1 times, such as about 1.5 times, the other of the positive and negative peak amplitudes.

In one set of embodiments, the electrical signal is a continuous waveform, for example a sine wave or square wave. Such a continuous waveform can be regarded as providing a continuously varying electrical signal.

In another set of embodiments, the electrical signal is a pulsed electrical signal. Such a pulsed electrical signal may result in a more gentle and controlled change of pH compared to, for example, the case of a non-varying voltage across the pair of electrodes.

In some embodiments, a duty cycle of the pulsed electrical signal is less than or equal to 90%, preferably less than or equal to 75%, most preferably less than or equal to 50%.

The duty cycle is the percentage of the ratio of pulse duration, or pulse width, to the total period of the waveform. The associated smaller total current "on" time associated with the pulsed electrical signal relative to a non-varying voltage across the pair of electrodes can assist to limit the progression of the above-mentioned low pH "front".

In some embodiments, the electrical (current or voltage) signal is both pulsed and bipolar. In such embodiments, a duty cycle associated with a positive component of the bipolar electrical signal may be the same as or different to a duty cycle associated with a negative component of the bipolar electrical signal.

In some embodiments, the at least one electrode of the one or more pairs of electrodes at which hydrogen ions are generated is insertable into the subject's oral cavity. In such embodiments, the aqueous solution may comprise the subject's saliva and/or one or more oral care agents.

Such oral care agent(s) may be at least one selected from a toothpaste, mouthrinse and a whitening agent.

It is noted, more generally, that the aqueous solution may, as well as the water, include one or more types of ions.

In some embodiments, the oral care system includes at least one cleaning element for mechanically and/or fluidically cleaning inside the subject's oral cavity.

In such embodiments, the dental tartar removal facilitated by the ions generation may be enhanced by the cleaning element(s).

Any suitable type of cleaning element can be contemplated. The at least one cleaning element may comprise bristles for brushing inside the subject's oral cavity, and/or a fluid-delivering nozzle for cleaning surfaces inside the subject's oral cavity. Alternatively or additionally, the at least one cleaning element may comprise a cup formed from a resilient material for rubbing against surfaces inside the subject's oral cavity. Such a cup may be, for instance, a so-called prophy cup.

In some embodiments, the one or more processors is or are configured to control movement of and/or fluid delivery from the at least one cleaning element.

Alternatively or additionally, the oral care system may comprise an actuator configured to cause movement of and/or fluid (i.e. liquid and/or gas) delivery from the at least one cleaning element.

Such an actuator may be controllable, e.g. by the processor(s), to implement a mechanical and/or fluidic treatment mode of the oral care system.

The actuator may, for example, include a drive train, e.g. a drive train including a motor, and/or a hydraulic or pneumatic pump.

In some embodiments, the one or more processors is or are configured to control movement of and/or fluid delivery from the at least one cleaning element based on the dental tartar indication.

In such embodiments, the one or more processors may be configured to control the actuator, based on the dental tartar indication, so that the actuator causes movement of and/or fluid delivery from the at least one cleaning element.

Thus, the mechanical and/or fluidic cleaning may be implemented, in addition to the electrochemical procedure, based on the dental tartar indication, for example via dental tartar-based control over a fluid pump, e.g. to control fluid jetting pressure, flow rate, volume and/or frequency; and/or control over a drive train, e.g. to control drive train characteristics, such as frequency, sweeping amplitude and/or the type of motion.

The type of motion adjustment may, for instance, involve adding a tapping motion ('jackhammer') of top of an oscillation, rotation or translation motion.

In such embodiments, the dental tartar indication-based actuator control may assist to remove electrochemically treated, e.g. softened, dental tartar more quickly, noting that the organic component of dental tartar may be less influenced by the electrochemical procedure than the inorganic component. In other words, the electrochemical procedure may assist to at least partially remove what can be regarded as a calcified calcium-phosphate scaffold, and the actuator control, e.g. implemented at the same time or subsequently to the electrochemical procedure, may assist to remove the organic material that is no longer or to a lesser extent held on dental piece(s) by the at least partially removed calcified calcium-phosphate scaffold.

Thus, the operation of the cleaning element(s) can be beneficially linked, together with the ion generation by the electrochemical system, to the dental tartar detection. This may facilitate oral treatment using the oral care device being implemented in a safe and effective manner.

In some embodiments, one or both of the electrodes is or are mechanically coupled to the actuator such that the movement of the actuator causes movement of the electrode(s).

In such embodiments, the actuator movement may result in an increased effective area, in other words footprint, in which the electrochemical treatment provided by the electrodes is implemented.

In some embodiments, the one or more processors is or are configured to control the electrochemical system to initiate generation of the ions based on the dental tartar indication being indicative of dental tartar being present at a location within the subject's oral cavity, and to terminate generation of the ions based on detection of enamel at the location.

Thus, the electrochemical treatment, e.g. via hydrogen ion generation, may be started in response to dental tartar being detected and stopped in response to enamel being detected at the same location. The latter may be due to dissolution of the dental tartar.

Alternatively or additionally, the processor(s) may be configured to adjust an electrical power used by the electrochemical system to generate the ions based on the dental tartar indication being indicative of an amount of dental tartar, such as an amount of dental tartar relative to enamel, present at said location within the subject's oral cavity.

Should, for example, the dental tartar indication be indicative of the dental tartar layer not exhibiting a diminished thickness, or a rate of decrease of the thickness of the dental tartar layer being equal to or below a threshold, during the electrochemical procedure, the processor(s) may be configured to increase the electrical power used by the electrochemical system to generate the ions and/or may issue a notification to the user, e.g. to the subject's dental care provider.

Thus, the oral care system may provide an electrochemical procedure that is adaptive to relatively stubborn dental tartar deposits.

In some embodiments, the electrical power (i.e., dose of electrochemical treatment) may be reduced as the tartar layer's thickness decreases. This may assist to protect enamel from any adverse impact associated with the electrochemical treatment.

In some embodiments, the oral care system comprises an oral care assembly that is insertable into the subject's oral cavity. In such embodiments, the oral care assembly may be included in, for example define, a brush head of a toothbrush.

In such embodiments, at least one ion generation electrode may be included in the oral care assembly.

Thus, the ions may be generated within the oral cavity, which may facilitate delivery of the ions within the oral cavity.

As an alternative or in addition to the at least one ion generation electrode included in the oral care assembly, detection element(s) of the detection system, e.g. detection elements comprising optical elements of the above-described optical detection system, may be included in the oral care assembly.

Including the detection element(s) in the oral care assembly, e.g. brush head, may provide a relatively straightforward way of arranging the detection system so as to permit dental tartar detection.

In some embodiments, the at least one ion generation electrode and the detection element(s) are included in the oral care assembly, e.g. brush head. This may provide a relatively straightforwardly implementable way of enabling combined dental tartar detection and ion delivery within the subject's oral cavity.

In some embodiments, the oral care system comprises an output device, and the one or more processors is or are configured to control the output device, based on the dental tartar indication, to provide a notification to a user.

In this manner, the user can be kept informed of the status of the subject's dental tartar.

In some embodiments, the notification comprises an alert to schedule a dental practitioner appointment, e.g. based on the dental tartar indication being indicative of dental tartar that is not removed following a number, such as a predetermined number, of treatments using the oral care system, or a time, e.g. brushing time, during which the oral care system has been used for treating the subject.

In such embodiments, the user may be at least one of the subject and the subject's dental care provider. In embodiments in which the dental care provider is notified via the notification, the notification may provide an alert for the dental care provider to schedule an appointment with the subject, e.g. for triaging purposes.

More generally, the oral care system may comprise, or be, a toothbrush, a mouthpiece, an irrigator, or a professional dental instrument operable by a dental practitioner or oral hygienist. Particular mention is made of an oral care system in the form of a toothbrush.

It is noted that oral care systems comprising, e.g. in the form of, a toothbrush, a mouthpiece, and an irrigator can be regarded as examples of a personal care oral system. Such personal care oral systems may be used by the subject him/herself in their own home.

According to another aspect there is provided a method of controlling an electrochemical system of an oral care system further comprising a detection system for detecting dental tartar within a subject's oral cavity, the method comprising: obtaining, via the detection system, a dental tartar indication indicative of dental tartar within the subject's oral cavity; and controlling, based on the dental tartar indication, the electrochemical system.

The ions, such as hydrogen ions, zinc ions, tin ions, copper ions, and/or silver ions, may be deliverable into the subject's oral cavity, as previously described. In some embodiments, the method does not include delivery of the ions within the subject's oral cavity, and correspondingly the method does not include any therapeutic treatment step.

According to a further aspect there is provided a computer program comprising computer program code which is configured, when the computer program is run on one or more processors included in an oral care system comprising a detection system for detecting dental tartar within a subject's oral cavity and an electrochemical system for generating ions, to cause the one or more processors to implement the method according to any of the embodiments disclosed herein.

One or more non-transitory computer readable media may be provided, which non-transitory computer readable media have a computer program stored thereon, with the computer program comprises computer program code which is configured, when the computer program is run on the one or more processors, to cause the one or more processors to implement the method according to any of the embodiments described herein.

More generally, embodiments described herein in relation to the method and computer program may be applicable to the oral care system, and embodiments described herein in relation to the oral care system may be applicable to the method and computer program.

These and other aspects of the invention will be apparent from and elucidated with reference to the embodiment(s) described hereinafter.

It is to be noted that the method and the computer program are not part of the claimed invention.

### BRIEF DESCRIPTION OF THE DRAWINGS

For a better understanding of the invention, and to show more clearly how it may be carried into effect, reference will now be made, by way of example only, to the accompanying drawings, in which:
FIG. 1 schematically depicts an oral care system according to a first example;
FIG. 2 schematically depicts an oral care assembly of the oral care system shown in FIG. 1 being used on a tooth with dental tartar;
FIG. 3 schematically depicts an oral care system according to a second example;
FIG. 4 schematically depicts an oral care system according to a third example;
FIG. 5 schematically depicts an oral care system according to a fourth example;
FIG. 6 provides a block diagram of an oral care system according to a fifth example;
FIG. 7 provides Fourier Transform Infrared (FTIR) diffuse reflectance spectra of hydroxyapatite (HAp) and octacalcium phosphate (OCP);
FIG. 8 provides FTIR specular reflectance spectra of HAp and OCP;
FIG. 9 provides first derivative FTIR diffuse reflectance spectra of HAp and OCP, expressed as raw reflection;
FIG. 10 provides a schematic depiction of dental tartar layered on a pellicle layer of a tooth;
FIG. 11 provides graphs showing solubility of two relevant crystalline phases of calcium phosphate as a function of pH;
FIG. 12 schematically depicts part of an oral care system according to a sixth example;
FIG. 13 schematically depicts part of an oral care system according to a seventh example;
FIG. 14 schematically depicts part of an oral care system according to an eighth example;
FIG. 15A shows a circuit for providing a unipolar pulsed electrical signal;
FIG. 15B shows a bipolar signal (upper part), and a unipolar pulsed electrical signal (lower part) provided by the circuit shown in FIG. 15A;
FIG. 16 schematically depicts actuator-driven movement of electrolysis electrodes according to an example;
FIG. 17 schematically depicts part of an oral care system according to a ninth example;
FIG. 18 shows part of an oral care system according to a tenth example;
FIG. 19 provides graphs of tartar and enamel dissolution depth in 30 seconds vs voltage across electrolysis electrodes;
FIG. 20 schematically depicts a 2D simulation setup including two electrolysis electrodes;
FIG. 21A provides a graph of a non-varying voltage;
FIG. 21B provides graphs of pH vs time at different points proximal to the electrodes shown in FIG. 20 when the electrical signal shown in FIG. 21A is provided to the electrodes;
FIG. 22A provides a graph of an electrical signal according to a first example;
FIG. 22B provides graphs of pH vs time at different points proximal to the electrodes shown in FIG. 20 when the electrical signal shown in FIG. 22A is provided to the electrodes;
FIG. 23A provides a graph of an electrical signal according to a second example;
FIG. 23B provides graphs of pH vs time at different points proximal to the electrodes shown in FIG. 20 when the electrical signal shown in FIG. 23A is provided to the electrodes;
FIG. 24 provides a graph of an electrical signal according to a third example;
FIG. 25 provides a graph of an electrical signal according to a fourth example;
FIG. 26 provides a flowchart of a method according to a first example, the method not being part of the claimed invention;
FIG. 27 provides a flowchart of a method according to a second example, the method not being part of the claimed invention; and
FIG. 28 provides a flowchart of a method according to a third example, the method not being part of the claimed invention.

### DETAILED DESCRIPTION OF THE EMBODIMENTS

The invention will be described with reference to the Figures. It should be understood that the Figures are merely schematic and are not drawn to scale. It should also be understood that the same reference numerals are used throughout the Figures to indicate the same or similar parts.

Provided is an oral care system comprising a detection system for detecting dental tartar within a subject's oral cavity, and an electrochemical system for generating ions for controlled delivery within the subject's oral cavity during an electrochemical procedure. One or more processors is or are configured to obtain, via the detection system, a dental tartar indication indicative of dental tartar within the subject's oral cavity, and control the electrochemical procedure provided by the electrochemical system based on the dental tartar indication. Further provided is a method of controlling an electrochemical system of such an oral care system, and a related computer program.

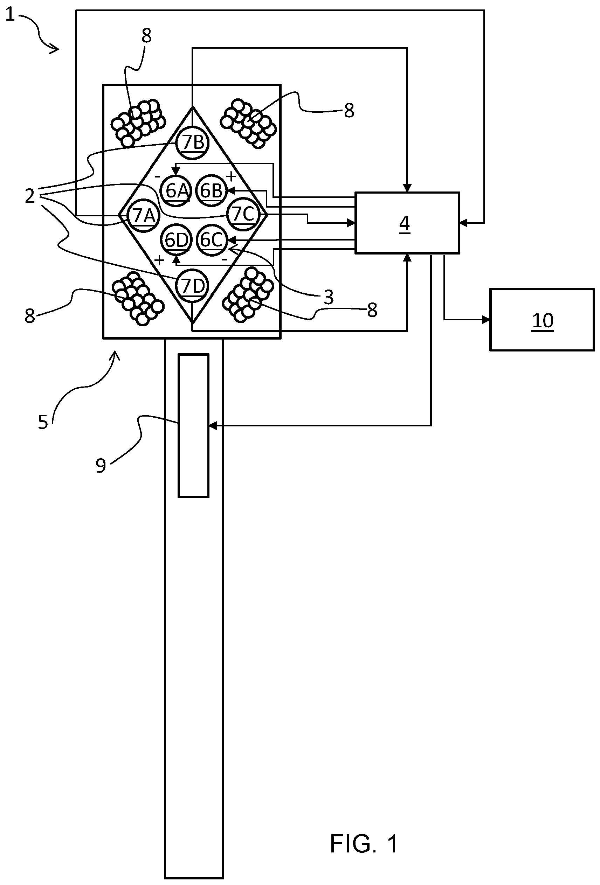

FIG. 1 schematically depicts an oral care system 1 according to an example. The oral care system 1 includes an electrochemical system 3 for generating ions for delivery within the subject's oral cavity (not visible in FIG. 1).

Such ions may be suitable for treating various dental tartar-related issues.

In some embodiments, the ions generated by the electrochemical system 3 are one or more selected from hydrogen ions, zinc ions, tin ions, copper ions, and silver ions.

Hydrogen ions may assist to lower a pH in the oral cavity proximal to dental pieces, e.g. teeth. Such lowering of the pH may assist in dissolution of dental tartar, as will be explained in more detail herein below.

In embodiments in which hydrogen ions are generated by the electrochemical system 3, the electrochemical system 3 may be arranged to generate the hydrogen ions via water, e.g. water in saliva, electrolysis, as also described in more detail herein below.

Zinc ions, tin ions, copper ions, and/or silver ions may provide various oral health benefits, including, for instance, antimicrobial action.

In embodiments in which zinc ions, tin ions, copper ions, and/or silver ions are generated by the electrochemical system 3, the electrochemical system 3 may include one or more zinc-comprising, tin-comprising, copper-comprising, and/or silver-comprising sacrificial electrodes.

More generally, the present disclosure is at least partly based on the insight that the presence, or otherwise, of dental tartar in the subject's oral cavity can beneficially be used as a guide for controlling the electrochemical system's 3 generation of ions.

In particular, when the electrochemical system 3 is configured to generate hydrogen ions, detection of dental tartar can justify lowering the pH proximal to the subject's dental pieces in order to dissolve the dental tartar. On the other hand, should no or only minimal quantities of dental tartar be detectable, it may instead be desirable to control the electrochemical system 3 so as to avoid lowering this pH, or even to increase the pH following a previous delivery of hydrogen ions, to minimize the risk of dissolution of healthy or intact enamel. This is because the enamel, in common with the dental tartar, may be more easily dissolvable at lower pH conditions.

In embodiments in which the electrochemical system 3 generates ions other than hydrogen ions, such as zinc ions, tin ions, copper ions, and/or silver ions, it may be desirable to restrict generation of such ions only to when dental tartar is detectable in the subject's oral cavity because of the dental tartar detection being indicative that ion treatment, e.g. antimicrobial ion treatment, is justified. On the other hand, should no or only minimal quantities of dental tartar be detectable, it may instead be desirable to control the electrochemical so as to avoid generation of such ions, for example to prolong the operating lifetime of the sacrificial electrodes from which the ions may be electrochemically generated.

To this end, the oral care system 1 comprises a detection system 2 for detecting dental tartar within the subject's oral cavity, and one or more processors 4. The processor(s) 4 is or are configured to obtain, via the detection system 2, a dental tartar indication indicative of dental tartar within the subject's oral cavity, and control the electrochemical system 3 to provide the electrochemical procedure, e.g. to electrochemically generate the ions and/or deliver the electrochemically generated ions within the subject's oral cavity, based on the dental tartar indication.

Dental tartar detection can, in particular, make lower pH-driven dental tartar dissolution safer to implement by assisting to lower the risk of uncontrolled and unwanted dissolution of healthy tooth-protecting enamel. This can mean that the oral care system 1 according to the present disclosure can be used for at-home tartar removal.

The detection system 2 may utilize any suitable dental tartar detection principle, for instance a detection principle that distinguishes one or more characteristics of dental tartar with respect to enamel.

For example, the detection system 2 may include an ion detection system configured to detect dental tartar, e.g. via a release rate of one or more ions from enamel being different from, e.g. lower than, a release rate of the one or more ions from dental tartar.

Such an ion release rate difference may reflect the fact that dental tartar is formed via transition from biofilm, which contains organic content, to calculus, which is mainly or even fully inorganic, via calcification and crosslinked network-forming processes.

In some embodiments, the detection system 2 may include a material hardness detection system configured to detect dental tartar via a hardness measurement, for example via a tapping element (not visible) configured to tap against materials in the subject's oral cavity.

In such embodiments, the detection system 2 may be configured to detect dental tartar based on a hardness or (viscoelastic) stiffness difference between dental tartar and enamel.

Dental tartar tends to be softer that enamel, particularly in its early phase of formation.

In some embodiments, the detection system 2 comprises an optical detection system for optically detecting dental tartar within the subject's oral cavity.

Optical detection of dental tartar has been found to be particularly advantageous at least partly owing to the differences between dental tartar's and enamel's interaction with light. This may be due to the different chemical/structural properties of dental tartar as compared to tooth enamel, as will be explained in more detail herein below.

In some embodiments, such as that shown in FIG. 1, the oral care system 1 comprises an oral care assembly 5 that is insertable into the subject's oral cavity. In such embodiments, the oral care assembly 5 may be included in, for example define, a brush head of a toothbrush. An example of this is shown in FIG. 1.

The electrochemical system 3 may comprise at least one ion generation electrode 6A, 6B; 6C, 6D, for instance water electrolysis electrode(s) and/or sacrificial electrode(s), for generating the ions for delivery within the subject's oral cavity.

In some embodiments, such as that shown in FIG. 1, the at least one ion generation electrode 6A, 6B; 6C, 6D is included in the oral care assembly 5.

Thus, the ions may be generated within the oral cavity, which may facilitate delivery of the ions within the oral cavity.

In embodiments in which the ions include hydrogen ions, at least one electrode 6A, 6B; 6C, 6D that generates the hydrogen ions may be insertable into the subject's oral cavity, with the aqueous solution comprising the water from which the hydrogen ions are generated via electrolysis comprising the subject's saliva and/or one or more oral care agents, such as toothpaste, mouthrinse and/or a whitening agent. This may provide a relatively physically simple way of providing a lower pH proximal to the subject's teeth.

In some embodiments, the electrochemical system 3 comprises a control arrangement (not visible in FIG. 1) and one or more pairs of electrodes 6A, 6B; 6C, 6D for contacting an aqueous solution, such as the subject's saliva and/or one or more oral care agents, such as toothpaste, mouthrinse and/or a whitening agent. In such embodiments, and as described in more detail herein below, the control arrangement may provide an electrical signal that causes at least one electrode of the one or more pairs of electrodes 6A, 6B; 6C, 6D to generate hydrogen ions from water in the aqueous solution.

In such embodiments, the processor(s) 4 may be configured to, based on the dental tartar indication indicating the presence of dental tartar, trigger the control arrangement to provide the electrical signal.

In embodiments in which the detection system 2 comprises, e.g. is in the form of, the optical detection system, the optical detection system may, for instance, comprise an imaging system for imaging the subject's oral cavity (e.g. including photodiode(s) and optical fiber(s)), and/or a non-imaging optical detection system.

In some embodiments, such as that shown in FIG. 1, the optical detection system 2 comprises at least one optical element 7A, 7B, 7C, 7D for emitting and/or receiving light. The at least one optical element 7A, 7B, 7C, 7D may be insertable into the subject's oral cavity.

The optical element(s) 7A, 7B, 7C, 7D may be of any suitable type, provided that they are able to emit and/or receive light to permit optical dental tartar detection.

In some embodiments, such as that shown in FIG. 1, the optical element(s) 7A, 7B, 7C, 7D comprise(s), for example is or are in the form of, optical fiber(s) arranged to emit and/or receive light within the subject's oral cavity. Other possibilities for the optical element(s) 7A, 7B, 7C, 7D can also be contemplated, as will be described in more detail herein below.

As an alternative or in addition to the at least one ion generation electrode 6A, 6B; 6C, 6D being included in the oral care assembly 5, the detection element(s), e.g. detection element(s) comprising, such as in the form of, optical element(s) 7A, 7B, 7C, 7D can be included in the oral care assembly 5. An example of this is shown in FIG. 1.

Including the optical element(s) 7A, 7B, 7C, 7D in the oral care assembly 5, e.g. brush head, may provide a relatively straightforward way of arranging the optical detection system 2 so as to permit dental tartar detection.

In some embodiments, such as that shown in FIG. 1, the at least one ion generation electrode 6A, 6B; 6C, 6D and the detection element(s), e.g. optical element(s) 7A, 7B, 7C, 7D, are included in the oral care assembly 5, e.g. brush head. Thus, the oral care assembly 5 can be regarded as a combined detection and treatment module, e.g. including optical illumination and sensing elements 7A, 7B, 7C, 7D together with treatment electrodes 6A, 6B; 6C, 6D.

This may provide a relatively straightforwardly implementable way of enabling dental tartar detection and ion delivery within the subject's oral cavity. In particular, an oral care assembly 5-comprising brush head may be insertable into the subject's oral cavity in order to enable the detection system 2 to detect whether or not dental tartar is present in the subject's oral cavity, and to facilitate delivery of ions generated by the electrochemical system 3 within the subject's oral cavity.

In some embodiments, the oral care system 1 comprises at least one cleaning element 8 for mechanically and/or fluidically cleaning inside the subject's oral cavity. In such embodiments, the dental tartar removal facilitated by the ions generation may be enhanced by the cleaning element(s) 8.

Any suitable type of cleaning element 8 can be contemplated. In the non-limiting example shown in FIG. 1, the at least one cleaning element 8 comprises bristles for brushing inside the subject's oral cavity. Alternatively or additionally, the at least one cleaning element 8 may comprise a cup formed from a resilient material for rubbing against surfaces inside the subject's oral cavity. Such a cup may be, for instance, a so-called prophy cup.

In some embodiments, such as that shown in FIG. 1, the oral care device 1 comprises an actuator 9 configured to cause movement of and/or fluid delivery from the at least one cleaning element 8.

In such embodiments, the one or more processors 4 may be configured to control the actuator 9, based on the dental tartar indication, so that the actuator 9 causes movement of and/or fluid delivery from the at least one cleaning element 8.

Thus, the mechanical and/or fluidic cleaning may be implemented, in addition to the electrochemical procedure, based on the dental tartar indication, for example via dental tartar-based control over a fluid pump, e.g. to control fluid jetting pressure, flow rate, volume and/or frequency; and/or control over a drive train, e.g. to control drive train characteristics, such as frequency, sweeping amplitude and/or the type of motion.

The type of motion adjustment may, for instance, involve adding a tapping motion ('jackhammer') of top of an oscillation, rotation or translation motion.

In such embodiments, the dental tartar indication-based actuator control may assist to remove electrochemically treated, e.g. softened, dental tartar more quickly, noting that the organic component of dental tartar may be less influenced by the electrochemical procedure than the inorganic component. In other words, the electrochemical procedure may assist to at least partially remove what can be regarded as a calcified calcium-phosphate scaffold, and the actuator control, e.g. implemented at the same time or subsequently to the electrochemical procedure, may assist to remove the organic material that is no longer or to a lesser extent held on dental piece(s) by the at least partially removed calcified calcium-phosphate scaffold.

Thus, the operation of the cleaning element(s) 8 can be beneficially linked, together with the ion generation by the electrochemical system 3, to the dental tartar detection. This may facilitate oral treatment using the oral care device 1 being implemented in a safe and effective manner.

In embodiments in which the cleaning element(s) 8 are arranged to be actuated by the actuator 9 to provide fluid delivery from the cleaning element(s), such fluid delivery may comprise delivery of a liquid within the oral cavity following delivery of hydrogen ions thereto. In other words, the processor(s) 4 may be configured to control the actuator 9 so that the actuator 9 causes liquid delivery from the cleaning element(s) 8 following the electrochemical system 3 being controlled to generate hydrogen ions for delivery within the subject's oral cavity.

Such delivery of liquid may assist to increase the pH proximal to the dental pieces following delivery of hydrogen ions. This may make for safer dental tartar dissolution via hydrogen ion delivery within the subject's oral cavity.

In a non-limiting illustrative example, and as shown in FIG. 1, the optical detection system 2 comprises photodiode(s) and optical elements 7A, 7B, 7C, 7D in the form of optical fibers arranged around the at least one pair of ion generation electrodes 6A, 6B; 6C, 6D. In the particular example shown in FIG. 1, the optical fibers and ion generation electrodes 6A, 6B; 6C, 6D are arranged in a diamond-shaped pattern in plan, as indicated by the parallelogram drawn around the circles representing the optical elements 7A, 7B, 7C, 7D and the circles representing the ion generation electrodes 6A, 6B; 6C, 6D.

More generally, the electrochemical system 3 may comprise a quadruple electrochemical electrode 6A, 6B; 6C, 6D configuration that is surrounded by four optical sensing elements 7A, 7B, 7C, 7D, e.g. based on optical fibers and photodiode(s), of the optical detection system 2.

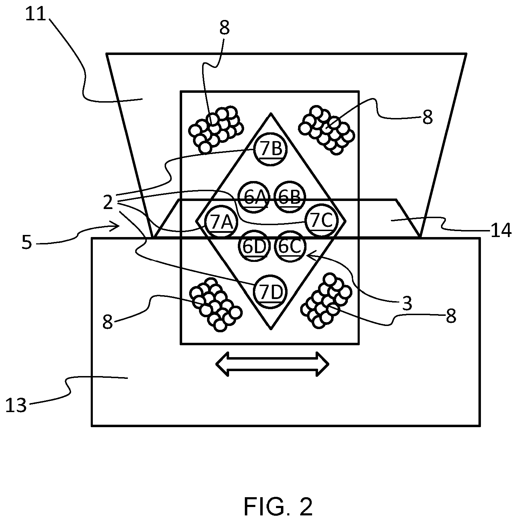

With reference to FIG. 2, such an arrangement may facilitate sensing of enamel 11 and dental tartar 14 by the detection system 2, e.g. the optical detection system, in particular during movement of the oral care assembly 5, e.g. brush head, inside the oral cavity. The gumline 13 is also schematically depicted in FIG. 2.

In some embodiments, such as that shown in FIGs. 1 and 2, two or more of the optical sensing elements 7A; 7B, 7D; 7C of the optical detection system 2 are each arranged in the oral care assembly 5, e.g. brush head, at respective lateral positions, and two or more of the optical sensing elements 7B; 7A, 7C; 7D are each arranged in the oral care assembly 5 at respective longitudinal positions, with the longitudinal direction of the oral care assembly 5 extending transversely relative to the lateral direction.

Such a quadruple arrangement of the optical sensing elements 7A, 7B, 7C, 7D may facilitate detection of dental tartar during lateral movement of the oral care assembly 5, as denoted in FIG. 2 by the double-headed arrow indicating lateral/horizontal motion, e.g. brush motion, and also during up-down longitudinal/vertical movements.

The arrangement of the four optical sensing elements 7A, 7B, 7C, 7D may permit differential, e.g. comparative, measurement of material inside the subject's oral cavity, and may correspondingly enable control over the electrochemical system 3, e.g. switching electrode signals, based on the material, for example dental tartar or enamel, and the horizontal or vertical motion of the oral care assembly 5, e.g. brush head.

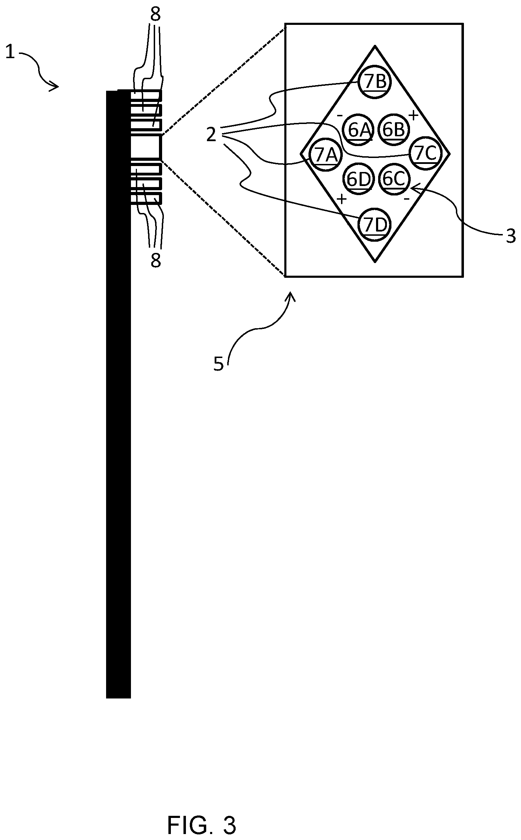

FIG. 3 schematically depicts an oral care system 1 having an oral care assembly 5 corresponding to that shown in FIGs. 1 and 2, but with a slightly different arrangement of cleaning elements 8, in this case bristles, with respect to the oral care assembly 5.

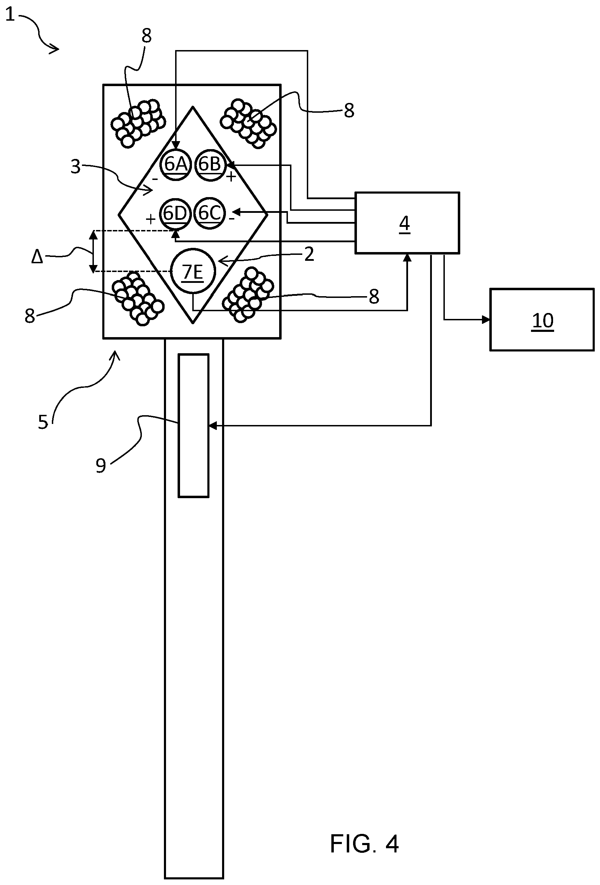

In some embodiments, such as that shown in FIG. 4, the optical detection system 2 comprises an optical element 7E comprising, e.g. in the form of, a spectral camera.

The spectral camera may be configured to image different wavelength windows, in other words bandwidths.

In such embodiments, a position of the spectral camera may be offset from position(s) of the ion generation electrode(s) 6A, 6B; 6C, 6D in the oral care assembly 5, e.g. offset from the one or two pairs 6A, 6B; 6C, 6D of ion generation electrodes 6A, 6B; 6C, 6D. This offset is denoted in FIG. 4 by a distance Δ from a periphery of closest electrode(s) 6D, 6C to the spectral camera and a center of the spectral camera's aperture.

Due to this offset Δ, an initial brushing session or brush stroke within a brushing session may, for example, be used to image the tooth to assess if dental tartar is present. If tartar is detected, then the electrochemical system 3 may be controlled to activate ion generation in a subsequent second session or stroke, when the brush reaches the corresponding location again.

Real-time location of the oral care assembly 5, e.g. brush head, sensing may be employed in such embodiments. To this end, the oral care assembly 5 may include one or more sensors (not visible), such as accelerometer(s) and/or inertial measurement unit(s), and the processor(s) 4 may be configured to predict the oral care assembly's 5 location based on data from the one or more sensors.

The processor(s) 4 may, for example, be configured to execute a machine-learning algorithm in order to enable real-time device location estimation and/or future location prediction or forecasting.

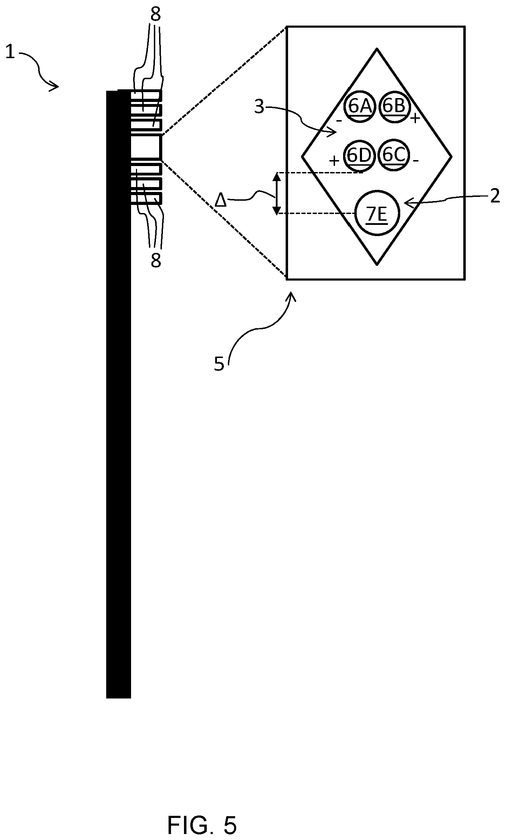

FIG. 5 schematically depicts an oral care system 1 having an oral care assembly 5 corresponding to that shown in FIG. 4, but with a slightly different arrangement of cleaning elements 8, in this case bristles, with respect to the oral care assembly 5.

It is noted that the oral care assemblies 5 shown in FIGs. 1 to 5 are not limited to being incorporated into toothbrushes, e.g. as or included in a brush head, and can alternatively be implemented in a full or partial mouthpiece or other types of oral care device such as combined brushing-flossing devices or others.

The oral care system 1 may comprise, or be, a toothbrush, a mouthpiece, an irrigator, or a professional dental instrument operable by a dental practitioner or oral hygienist. Particular mention is made of an oral care system 1 in the form of a toothbrush.

It is noted that oral care systems 1 comprising, e.g. in the form of, a toothbrush, a mouthpiece, and an irrigator can be regarded as examples of a personal care oral system 1. Such personal care oral systems 1 may be used by the subject him/herself in their own home.

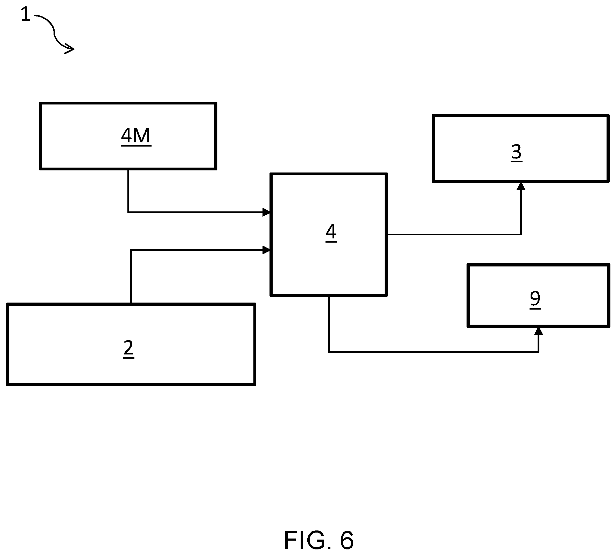

FIG. 6 provides a block diagram of an oral care system 1. The oral care system 1 includes the detection system 2, the electrochemical system 3, and the actuator 9 described above.

The actuator 9 may be controllable to implement a mechanical and/or fluidic cleaning mode, e.g. via drive train motion, as previously described.

In embodiments in which the detection system comprises, or is in the form of the optical detection system 2, the optical detection system 2 may include an optical spectrum module, e.g. comprising a light source and optical detector, and/or a spectral camera or other optics, such as optical fibers, to acquire spectral information.

In some embodiments, the optical detection system 2 comprises a light source, for example a source of infrared wavelength light, for instance mid infrared light emitting diode(s), e.g. for providing a spectral range of 2.7 to 4.7 µm. Alternatively or additionally, the optical detection system 2 may include optical fiber(s)/waveguide(s), sensor element(s), e.g. to detect reflected infrared light, such as photodiode(s), and/or a spectral camera, such as a hyper-spectral camera. Such a spectral camera may, for instance, include a complementary metal oxide semiconductor (CMOS) sensing element.

The electrochemical system 3 may be regarded as being or comprising an electrochemical treatment unit or cell comprising ion generation electrode(s) 6A, 6B; 6C, 6D.

The processor(s) 4 may include, e.g. be in the form of, a microcontroller configured to obtain, via the detection system 2, a dental tartar indication indicative of dental tartar within the subject's oral cavity, and control the electrochemical system 3 to provide electrochemical treatment, e.g. to electrochemically generate the ions, based on the dental tartar indication.

The processor(s) 4 may also be configured to control the actuator 9 so that the oral care system 1 operates in a mechanical and/or fluidic cleaning mode while the electrochemical system 3 is being controlled (optionally electrochemical system 3 and actuator 9).

In some embodiments, such as those shown in FIGs. 1 and 4, the oral care system 1 comprises an output device 10, with the processor(s) 4 being configured to control the output device 10, based on the dental tartar indication, to provide a notification to a user.

In this manner, the user can be kept informed of the status of the subject's dental tartar.

In some embodiments, the notification comprises an alert to schedule a dental practitioner appointment, e.g. based on the dental tartar indication being indicative of dental tartar that is not removed following a number, such as a predetermined number, of treatments using the oral care system 1, or a time, e.g. brushing time, during which the oral care system 1 has been used for treating the subject.

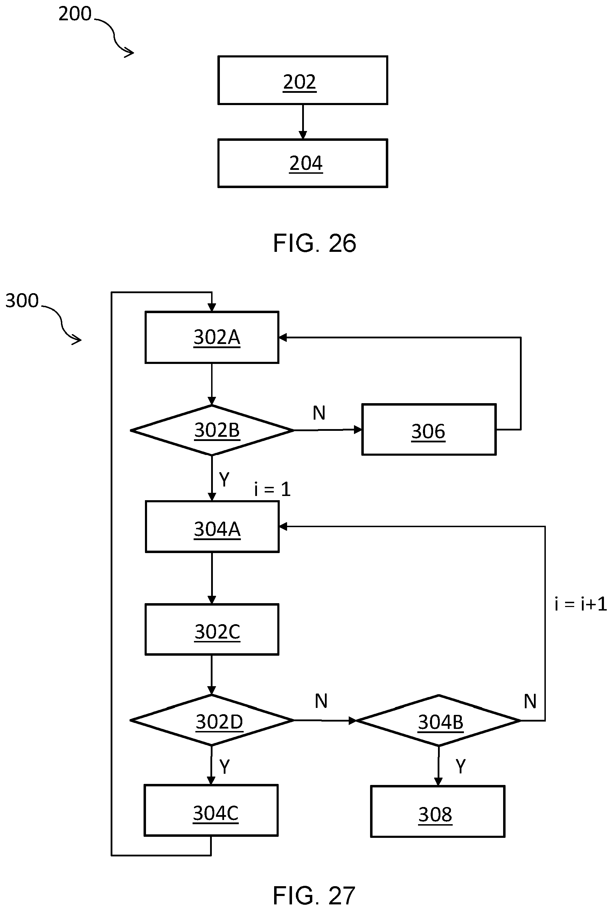

In such embodiments, the user may be at least one of the subject and the subject's dental care provider. In embodiments in which the dental care provider is notified via the notification, the notification may provide an alert for the dental care provider to schedule an appointment with the subject, e.g. for triaging purposes. An example of this will be described in more detail herein below with reference to FIG. 27.

In some embodiments, the processor(s) 4 is or are configured to execute a spectral comparison algorithm to distinguish between dental tartar and enamel, with the dental tartar indication comprising a result of the spectral comparison algorithm.

In other words, the spectral comparison algorithm may run on the processor(s) 4 and the result of the spectral comparison algorithm may be used to generate control signals for controlling the electrochemical system 3. Such control signals may, for example, initiate or terminate electrochemical treatment, e.g. ion generation, based on the result of the spectral comparison algorithm.

An electrochemical mode of the oral care system 1 can be activated or deactivated based on the result of the spectral comparison algorithm.

In some embodiments, such as that shown in FIG. 6, the oral care system 1 includes spectral input 4M for running the spectral comparison algorithm. The spectral input 4M can, for instance, be stored in a local memory included in an oral care device, e.g. toothbrush, comprising the oral care system 1, and/or in a cloud-based server.

The detection system 2 can detect the dental tartar in any suitable manner.

In some embodiments, the detection system 2 comprises, e.g. is in the form of, a spectral detection system configured to spectroscopically detect the presence of dental tartar within the subject's oral cavity. Thus, the dental tartar indication may be based on spectroscopic detection of the presence of dental tartar.

Such spectroscopic detection of the presence of dental tartar may capitalize on the different chemical/structural properties of dental tartar compared to enamel resulting in spectral differences between these materials that can be spectroscopically discerned. A key point of the present disclosure concerns use of spectral information to distinguish between dental tartar and enamel.

In some embodiments, the spectroscopic detection of the presence of dental tartar within the subject's oral cavity comprises infrared spectroscopic detection. Infrared spectroscopic detection, e.g. using mid and/or near infrared radiation, may provide a relatively convenient and reliable way of distinguishing between enamel and dental tartar.

In such embodiments, the electrochemical system 3 may be adaptively controlled, e.g. by switching the electrochemical treatment on or off, based on infrared optical measurement of material within the oral cavity.

When the electrochemical treatment comprises generating hydrogen ions for delivery within the subject's oral cavity, infrared optical measurement-based control may provide a particularly convenient and reliable way of assisting to minimize the risk of uncontrolled/unwanted dissolution of healthy enamel.

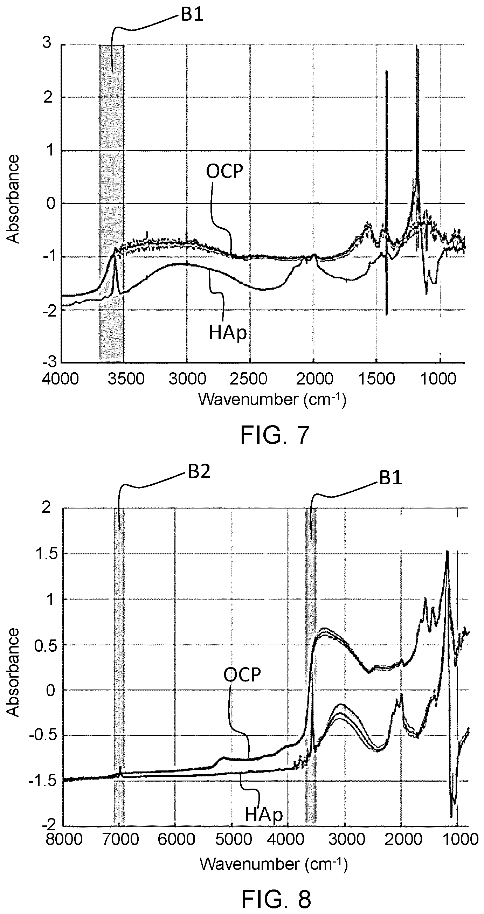

To illustrate the above-mentioned infrared spectroscopic detection, FIG. 7 provides FTIR diffuse reflectance spectra of hydroxyapatite (HAp) and octacalcium phosphate (OCP). HAp is a component of enamel and OCP is major inorganic constituent of early dental tartar.

Band B1 in FIG. 7 is a 3500-3600 cm⁻¹ band that provides a significant spectral difference, noting a defined peak being present for HAp but not for OCP, between HAp and OCP.

Evident in FIG. 8, which provides FTIR specular reflectance spectra of HAp and OCP, are overtones of this band at about 7000 cm⁻¹ (see band B2 in FIG. 8) which can also be used for enamel vs dental tartar differentiation.

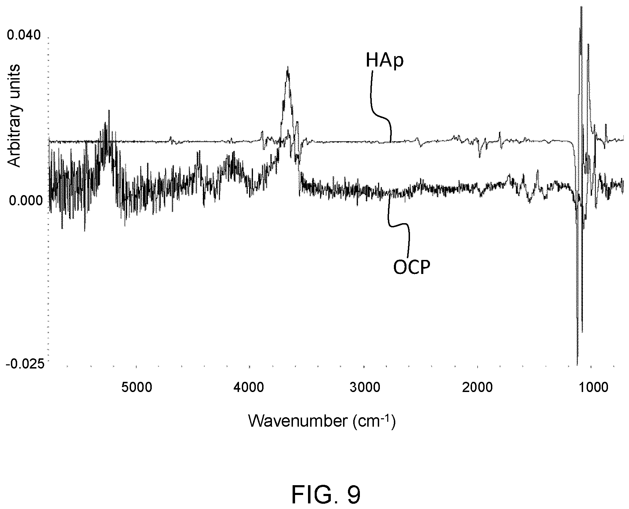

Next to looking into absolute reflectance/absorbance signals, first or second derivative spectra can also be assessed, and/or a ratio of spectral bands to better differentiate between enamel and dental tartar. FIG. 9 provides first derivative FTIR diffuse reflectance spectra of HAp and OCP, expressed as raw reflection.

In some embodiments, the optical detection system 2 comprises a combination of light emitting diodes and a photodiode whose arrangement capitalizes on the relatively low reflectivity of dental tartar in the above-mentioned wavelength windows.

Light from a 2.8 or 1.4 µm wavelength light emitting diode (3600 or 7200 cm⁻¹) may be normalized with a background reflection from a 2.4 µm wavelength light emitting diode (4200 cm⁻¹).

More generally, the spectral measurement may be implemented using an infrared source, a Bragg's grating and an infrared detector. It is noted that a camera may, for instance, be employed as the infrared detector.

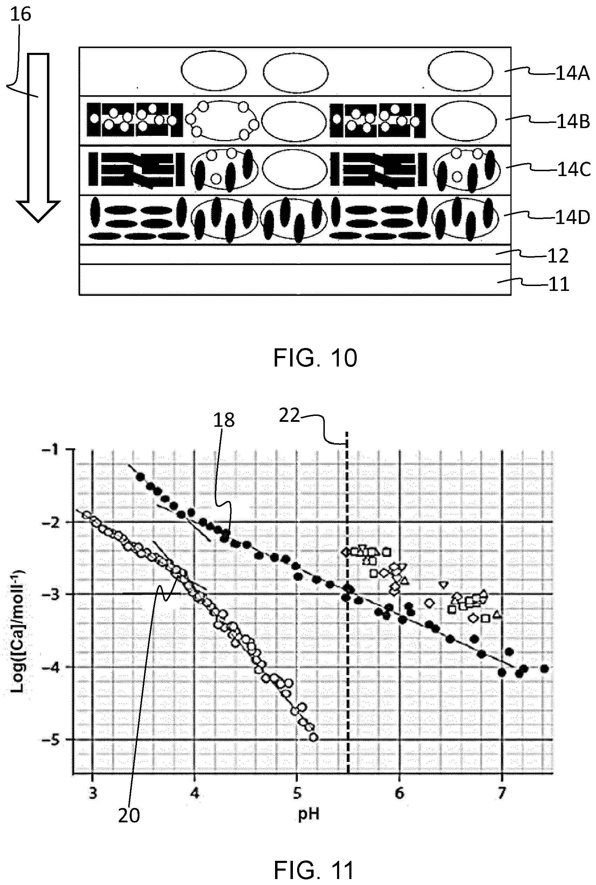

FIG. 10 schematically depicts an enamel layer 11 of a tooth, and a pellicle layer 12 on the enamel layer 11. FIG. 10 also shows dental tartar 14A, 14B, 14C, 14D layered on the pellicle layer 12.

The dental tartar 14A-D shown in FIG. 10 includes several different calcium phosphate crystalline phases which have different aqueous solubilities. The dental tartar layer 14A furthest from the pellicle layer 12 comprises dicalcium phosphate dihydrate (DCPD), the layer 14B adjacent the dental tartar layer 14A comprises octacalcium phosphate (OCP), the layer 14D closest to the pellicle layer 12 and the layer 14C between the layer 14B and the layer 14D may comprise a relatively small quantity of hydroxyapatite (HAp). HAp is a key component of the enamel layer 11, as previously described.

HAp has lower aqueous solubility than OCP, and OCP has lower aqueous solubility than DCPD, such that the relative and/or absolute aqueous solubility decreases in the direction of arrow 16. The absolute solubility of OCP and DCPD in water, is however low, and their proximity to the enamel layer 11, can therefore hamper removal of dental tartar, particularly by a subject in their own home.

The aqueous solubilities of the above-mentioned crystalline phases have been found to be pH dependent. In particular, dissolution in aqueous solution can be assisted by lowering the pH. FIG. 11 provides graphs 18, 20 showing solubility of tartar-comprising crystalline phases of calcium phosphate as a function of pH. FIG. 11 generally shows that the concentration of calcium ions dissolved in solution increases as the pH decreases for both OCP (graph 18), and HAp (graph 20); at pH 5.5 the solubility of OCP being almost 2 orders of magnitude higher than HAp.

A pH at which dental enamel 11 may start to dissolve is also denoted by the dotted line 22 in FIG. 11. This pH may be around pH 5.5, clinically considered the critical in-mouth pH at which dental enamel dissolves (clinically accepted as a threshold).

The present disclosure is partly based on the realization that by controlling the pH local to the teeth to be less than 7 but equal to or greater than 5.5, the OCP and DCPD of dental tartar 14, in particular early stage dental tartar, can be dissolved but with minimal or no damage to dental enamel 11. This is because such a pH range may minimize dissolution of HAp in the dental enamel 11, as shown in FIG. 11.

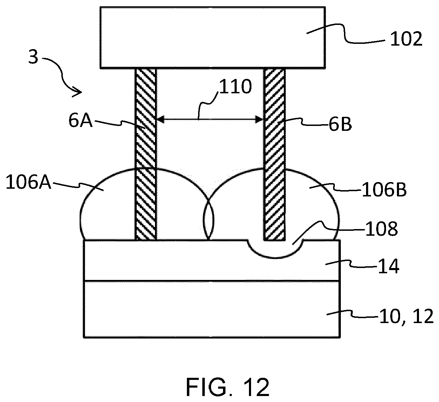

FIG. 12 schematically depicts an electrochemical system 3 according to an example. A control arrangement 102 is configured to provide an electrical signal. The electrochemical system 3 also comprises a pair of electrodes 6A, 6B for contacting an aqueous solution. The pair of electrodes 6A, 6B are each connected to the control arrangement 102 and configured to, responsive to the electrical signal, generate hydrogen ions from water in the aqueous solution.

Implicit in this generation of hydrogen ions, in other words H⁺ ions, from water in the aqueous solution is that a potential difference between the electrodes 6A, 6B resulting from the electrical signal is sufficient for water electrolysis to take place.

The electrodes 6A, 6B can be made of any suitable electrically conductive material capable of electrochemical generation of hydrogen ions via water electrolysis. For example, the electrodes 6A, 6B may be selected from platinum electrodes, platinum-clad electrodes, such as platinum clad titanium electrodes, gold electrodes, gold-clad electrodes, carbon electrodes, such a graphite electrodes, and polymer-based electrodes in which the polymer is embedded with the conductive material, for example with a conductive material selected from platinum, gold, or carbon, e.g. graphite.

Each of the pair of electrodes 6A, 6B may be a non-sacrificial electrode. The term "non-sacrificial electrode" may mean that the electrodes 6A, 6B are each formed of a conductive material which does not, or only negligibly, dissociates into ions in response to the electrical signal.

Referring again to FIG. 12, the following reaction may occur at the electrode 6A when this electrode is operated as a cathode: 2H₂O + 2e⁻ ↔ 2OH⁻ + H₂. (or, 2H⁺+ 2e⁻ ↔ H₂)

This production of hydroxide ions (or consumption of hydrogen ions) may raise the pH proximal to the electrode 6A. Such a local increase in pH, denoted in FIG. 12 by area 106A around electrode 6A, may have less influence on dental tartar 14 solubility than the local decrease in pH proximal to the electrode 6B, denoted by area 106B around electrode 6B, when the latter is operated as an anode: H₂O ↔ 2H⁺ + 0.5O₂ + 2e⁻ (or, 2OH⁻ ↔ O₂ + 2H⁺ + 2e⁻). This is because dental tartar dissolution, as represented in FIG. 12 by a tartar dissolution spot 108 proximal to the electrode 6B, may be assisted by lowering the pH, in other words increasing the hydrogen ion concentration, as previously explained.

The pair of electrodes 6A, 6B may be spaced apart from each other by any suitable distance 110, such as a distance 110 greater than 50 µm, for example between 50 µm and 1 mm.

A distance 110 greater than 50 µm may assist to suppress immediate neutralization of the hydrogen ions generated at the anode by the hydroxide ions generated at the cathode.

A half-distance (L/2) of, for instance, 25 µm may be covered by the pH front in ~0.07 s (L²/4D, where D = 9×10⁻⁹ m²/s, the diffusion coefficient of H⁺ ions). This lower limit of 50 µm may mean that a total treatment time is >0.07 s. This total treatment time is the time period, e.g. predetermined time period, during which the electrical signal is provided to the pair of electrodes 6A, 6B (and so is not the current on/off time in a pulse in embodiments in which the electrical signal is pulsed).

More generally, it has been found that employing a non-varying voltage across the pair of electrodes 6A, 6B provided by a direct current source can provide relatively uncontrolled generation of hydrogen ions. An electrical signal, for example time-varying electrical signal can, by contrast, assist to enhance the control that can be exerted over the generation of hydrogen ions, and thus the local pH to which the subject's teeth are exposed. This is explained in more detail herein below.

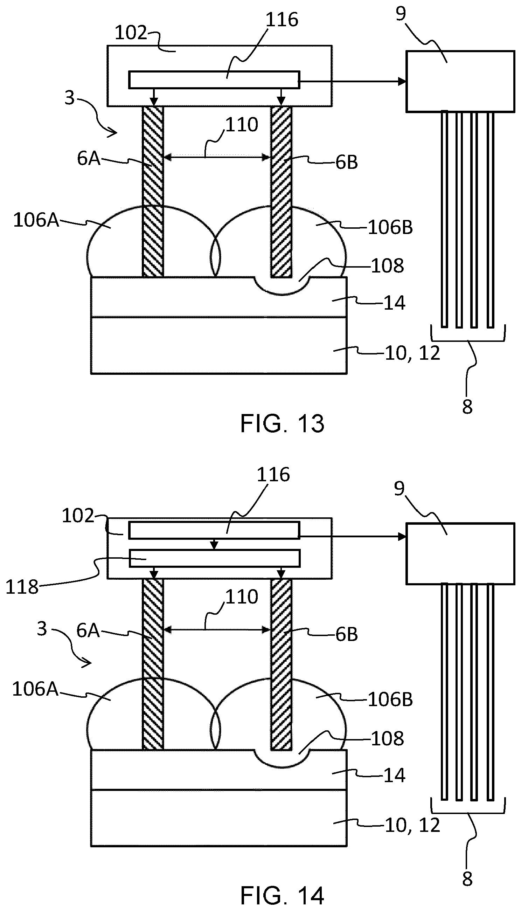

In some embodiments, such as that shown in FIG. 13, the oral care system 1 comprises an actuator 9 connected to the control arrangement 102. In such embodiments, the actuator 9 may be configured to move in accordance with the electrical signal generated by the control arrangement 102.

The term "move in accordance with the electrical signal" may mean in this context that an actuator control signal provided by the control arrangement 102 to control the movement of the actuator 9 and the electrical signal are the same as each other, or one of the actuator control signal and the electrical signal is based on the other of the actuator control signal and the electrical signal.

The actuator control signal may, for example, be in phase with the electrical signal, or a phase difference may be defined between the actuator control signal and the electrical signal.

The movement of the actuator 9, for example causing movement of an oral care assembly 5, e.g. brush head, may thus be provided alongside the hydrogen ion generation, with concomitant tartar removal enhancement.

Movement of the actuator 9 in accordance with the electrical signal provided to the electrodes 6A, 6B may, in certain embodiments, facilitate a particular cleaning movement, for example a reciprocating movement, being provided by the actuator 9, in addition to the electrical signal assisting to control hydrogen ion generation at the pair of electrodes 6A, 6B.

Any suitable type of actuator 9 can be contemplated, for example a motor, such as a reciprocating motor (not visible in the Figures).

In some embodiments, such as that shown in FIG. 13, the system 1 comprises at least one cleaning element 8 for mechanically cleaning inside the oral cavity, which at least one cleaning element 8 is mechanically coupled to the actuator 9 such that the movement of the actuator 9 causes movement of the at least one cleaning element 8.

Any suitable type of cleaning element 8 can be contemplated. In the non-limiting example shown in FIG. 13, the at least one cleaning element 8 comprises bristles for brushing inside the subject's oral cavity. In such an embodiment, the dental tartar removal facilitated by the hydrogen ions generation may be enhanced by the bristles movement resulting from the movement of the actuator 9 in accordance with the electrical signal.

A reciprocating movement of the actuator 9, e.g. the above-mentioned reciprocating motor, may be provided in accordance with a periodically varying electrical signal.

In such embodiments, the reciprocating movement of the actuator 9 may cause the cleaning element(s) 8, for example bristles, to correspondingly move in a reciprocating manner.

Such a periodic electrical signal may also assist to control the local pH, in particular so as to help reduce the risk of the local pH at one or both of the electrodes 6A, 6B from decreasing below a certain pH level, as will be explained in more detail herein below.

This combination of mechanical dental tartar break-up and low local pH-assisted dissolution of the dental tartar may be particularly effective, and may, for instance, also benefit from being implementable by the subject using the system 1 in their own home.

In certain embodiments, such as that shown in FIG. 13, the control arrangement 102 includes drive train circuitry 116 configured to provide the actuator control signal to control the actuator 9 and provide the electrical signal to the pair of electrodes 6A, 6B.

In such embodiments, the actuator control signal may be the same as the electrical signal, so as to provide an advantageously simple and cost-effective electrical design.

In alternative embodiments, such as that shown in FIG. 14, the control arrangement 102 comprises, in addition to the drive train circuitry 116 configured to provide the actuator control signal, electrode control circuitry 118 configured to convert the drive train control signal to the electrical signal for providing to the pair of electrodes 6A, 6B.

The electrode control circuitry 118 can, for example, include particularly only passive electronic components, such as diode(s), resistor(s) and/or capacitor(s), arranged to convert the drive train control signal to the electrical signal.

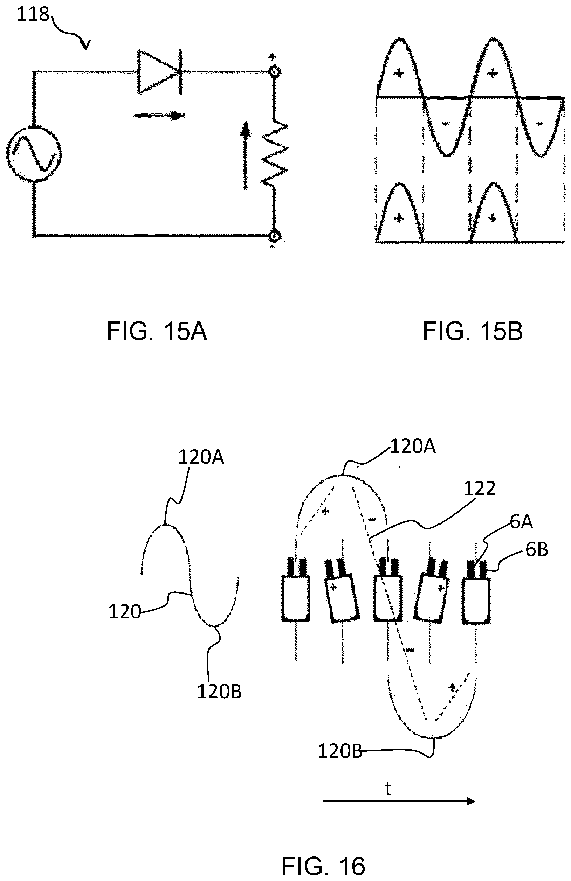

An example of such electrode control circuitry 118 is shown in FIG. 15A. In this example, the diode converts the alternating current signal shown in the upper part of FIG. 15B to a rectified output waveform, in which the negative half-cycle is absent, as shown in the lower part of FIG. 15B.

The drive train circuitry 116 can be implemented in any suitable manner, such as by the drive train circuitry 116 comprising a microcontroller, e.g. motor microcontroller, configured to generate the actuator control signal.

It is also noted that the alternating current signal of the example shown in FIGs. 15A and 15B need not be provided via drive train circuitry 116, and in other examples may be provided by a power supply, for example, a mains power source (not visible in the Figures) arranged to power the system 1.

In such examples, the control arrangement 102 may include the electrode control circuitry 118 to provide a unipolar pulsed signal (as shown in FIG. 15B, lower part) from an alternating current signal provided by the power supply, with the drive train control circuitry 116 being omitted.

In some embodiments, such as that shown in FIG. 16, one or both of the electrodes 6A, 6B is or are mechanically coupled to the actuator 9 such that the movement of the actuator 9 causes movement of the electrode(s) 6A, 6B.

In the non-limiting example shown in FIG. 16, the electrical signal 120 is a periodic bipolar electrical signal varying with time, t.

In a positive polarity half-cycle 120A of the electrical signal 120 shown in FIG. 7, the actuator 9, and thus the electrodes 6A, 6B, exhibit a first movement. In a negative polarity half-cycle 120B of the electrical signal 120, the actuator 9, and thus the electrodes 6A, 6B, exhibit a second movement mirroring the first movement. The gradient/slope of the electrical signal 120 is represented in FIG. 16 by the dotted lines 122.

This may be implemented by the control arrangement 102 providing an actuator control signal to control the actuator 9 that is in phase with the electrical signal provided to the electrodes 6A, 6B.

The actuator movement may result in an increased effective area, in other words footprint, in which the electrochemical treatment provided by the electrodes 6A, 6B is implemented. Moreover, the alternating polarity may mean that the hydrogen ion generation is implemented at both of the electrodes 6A, 6B but sequentially, thereby further assisting to increase the effective area in which the electrochemical treatment is provided. In addition, the periodic electrical signal may also assist to control the local pH, in particular so as to reduce the risk of the local pH at the electrodes 6A, 6B decreasing below a certain pH level, as will be explained in more detail herein below.

In alternative embodiments, the control arrangement 102 may be configured to provide an actuator control signal to control the actuator 9, with a phase difference being defined between the actuator control signal and the electrical signal provided to the electrodes 6A, 6B.

Thus, the electrical signal can be desynchronized from the actuator control signal, and thus desynchronized from movement of the actuator 9, e.g. brush head motion, such as brush head reciprocation.

Such a phase difference can be introduced in any suitable manner, such as via one or more capacitor(s), inductor(s), etc. Thus, a relatively small number of electrical components (in addition to those for providing the actuator control signal) may be required to provide the phase difference.

In some embodiments, the control arrangement 102 comprises, in addition to drive train circuitry 116 configured to provide the actuator control signal, electrode control circuitry 118 configured to convert the actuator control signal to the electrical signal provided to the electrodes 6A, 6B such that the phase difference is deliberately defined between the actuator control signal and the electrical signal.

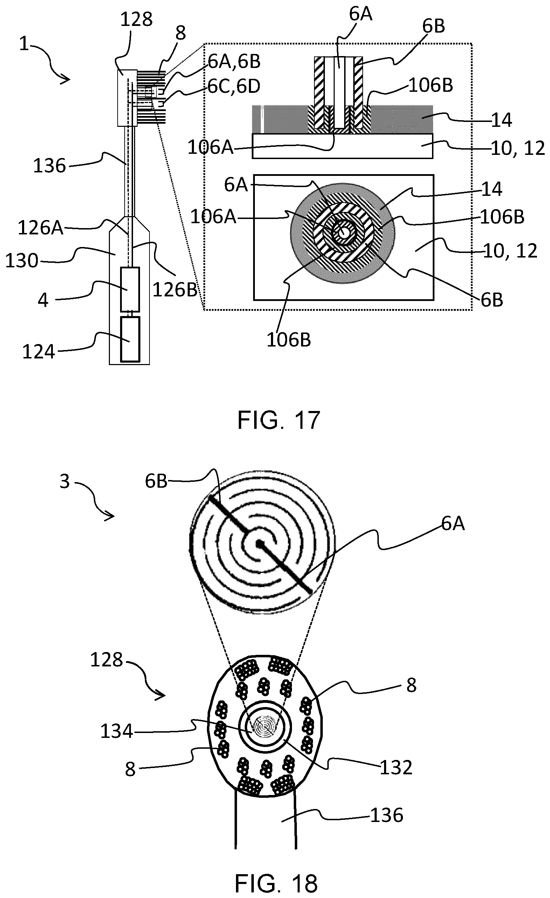

In some embodiments, such as that shown in FIG. 17, the oral care system 1 comprises a plurality of pairs of electrodes 6A, 6B; 6C, 6D. In such embodiments, the control arrangement 102 may be configured to provide the electrical signal to each of the plurality of pairs of electrodes 6A, 6B; 6C, 6D.

The oral care system 1 may be powered in any suitable manner, such as via a mains source of electricity and/or by one or more batteries 124. In embodiments in which the oral care system 1 is powered by one or more batteries 124, the control arrangement 102 may be configured to generate the electrical signal, for example a current or voltage signal, from a direct current resulting from the one or more batteries 124.

A first electrical connection 126A may connect the control arrangement 102 to one of the electrodes 6A of the pair of electrodes 6A, 6B, with a second electrical connection 126B connecting the control arrangement 102 to the other electrode 6B of the pair of electrodes 6A, 6B.

In the non-limiting example shown in FIG. 17 in which two pairs of electrodes 6A, 6B; 6C, 6D are included in the oral care system 1, the first electrical connection 126A connects the control arrangement 102 to the electrodes 6A and 6C, and the second electrical connection 126B connects the control arrangement 102 to the electrodes 6B and 6D.

In some embodiments, such as that shown in FIG. 17 the oral care system 1 comprises a toothbrush including a brush head 128 attached to a handle 130. In such embodiments, the pair of electrodes 6A, 6B are preferably included in the brush head 128, with the control arrangement 102, e.g. together with the processor(s) 4, being included in the handle 130.

In the non-limiting example shown in FIG. 17, the one or more batteries 124 are housed, together with the processor(s) 4 and/or the control arrangement 102, within the handle 130.

In some embodiments, the brush head 128, including the pair of electrodes 6A, 6B, is detachably coupled to the handle 130 to enable replacement of the brush head 128 without having to replace the control arrangement 102 included in the handle 130. In such embodiments, the first and second electrical connections 126A, 126B between the control arrangement 102 included in the handle 130 and the pair of electrodes 6A, 6B included in the (replacement) brush head 128 may be established at the same time as the brush head 128 is attached, for example snap-fitted or push-fitted, to the handle 130.

In the non-limiting example shown in FIG. 17, each of the plurality of pairs of electrodes 6A, 6B; 6C, 6D are included in the brush head 128.

In some embodiments, such as that shown in FIG. 17, the at least one cleaning element 8 comprises bristles, and the bristles extend further from the brush head 128 than each of the pair of electrodes 6A, 6B, such that pair of electrodes 6A, 6B are both recessed relative to the bristles.

The at least one cleaning element 8, and in some cases the brush head 128 as a whole, may be moved by an actuator 9, as previously described.

In embodiments, in which the at least one cleaning element 8 comprises the cup formed from a resilient material, one or both of the pair of electrodes 6A, 6B may be mounted inside the cup.

In embodiments, such as that shown in FIG. 17, in which a plurality of pairs of electrodes 6A, 6B; 6C, 6D is included in the oral care system 1, the oral care system 1 may include a plurality of cups. In such embodiments, one or both electrodes 6A, 6B; 6C, 6D of each pair may be mounted inside a respective cup of the plurality of cups.

The electrodes 6A, 6B can be arranged relative to each other in any suitable manner. In some embodiments, the electrodes 6A, 6B are arranged with one of the electrodes 6B defining an outer electrode 6B which at least partially surrounds the other of the electrodes 6A that defines an inner electrode 6A. An example of this is shown in FIG. 17.

FIG. 17 also shows the area 106A around the electrode 6A, and the area 106B around the electrode 6B. In one of these areas 106A, 106B, depending on the electrical signal, the local pH may be increased due to hydroxide ion generation while in the other of these areas 106B, 106A the local pH may be decreased due to hydrogen ion generation, as previously described in relation to FIG. 12.

As an alternative or in addition to one of the electrodes 6B defining an outer electrode 6B which at least partially surrounds an inner electrode 6A, the pair of electrodes 6A, 6B may be interdigitated.

The term "interdigitated" in this context may mean that each of the electrodes 6A, 6B has a plurality of electrode members spaced apart from each other, with electrode members of one of the pair of electrodes 6A, 6B being arranged in spaces between electrode members of the other of the pair of electrodes 6B, 6A.

This may provide a particularly spatially efficient arrangement of the pair of electrodes 6A, 6B, particularly when the pair of electrodes 6A, 6B is to be included in a brush head 128 and/or mounted within a cup where space may be limited.

A non-limiting example of such an interdigitated arrangement of the pair of electrodes 6A, 6B is depicted in FIG. 18.

Similarly to the non-limiting example shown in FIG. 17, cleaning elements 8 comprising bristles are included in the oral care system 1 shown in FIG. 18. In the latter non-limiting example, a plurality of peripheral tufts of bristles are arranged around a centrally positioned pair of interdigitated electrodes 6A, 6B.

In some embodiments, such as that shown in FIG. 18, the oral care system 1 comprises a cup 132 for inserting into the subject's oral cavity, with the cup 132 having a recess 134 in which one or both of the pair of electrodes 6A, 6B is or are mounted. In this manner, the cup 132 may assist to protect the pair of electrodes 6A, 6B and minimize the risk of the pair of electrodes 6A, 6B unintentionally contacting tissue within the subject's oral cavity.

The cup 132 may be formed of any suitable, for example electrically insulating, material. In some embodiments, the cup 132 is formed from a resilient material for rubbing against surfaces inside the subject's oral cavity, as previously described.

The cup 132 formed from the resilient material may flare and flex to contours of the subject's teeth to aid in selective stain removal and cleaning between teeth.

Any suitable resilient material can be considered for the cup 132, such as silicone rubber.

In some embodiments, such as that shown in FIGs. 17 and 18, the brush head 128 is attached, for example detachably coupled, to a handle 130 of the oral care system 1, in this case in the form of a toothbrush, via a neck 136. Such a neck 136 may extend between an upper end of the handle 130 and a lower end of the brush head 128.

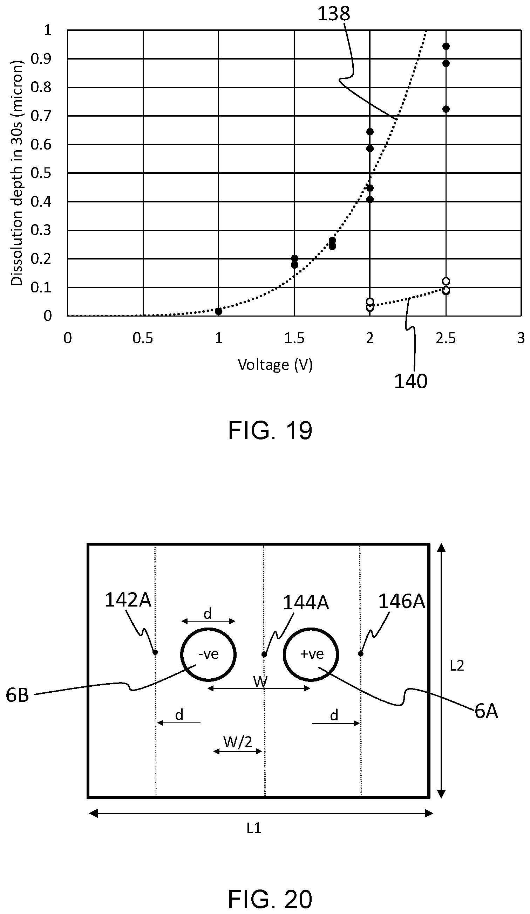

FIG. 19 provides a graph 138 of tartar dissolution depth in 30 seconds vs continuous DC voltage across the pair of electrodes 6A, 6B. As the voltage increases, the associated local decrease in pH caused by the hydrogen ion generation results in dissolution of dental tartar to a greater depth. However, FIG. 19 also provides a graph 140 of dental enamel dissolution depth in 30 seconds vs continuous DC voltage across the pair of electrodes 6A, 6B which shows that relatively low pH conditions caused by relatively high voltages can cause dissolution of dental enamel.

FIG. 20 schematically depicts a 2D simulation setup including two electrolysis electrodes 6A, 6B in a 0.1 M KCl solution (to correspond to experimental measurements, shown in Fig. 10. Saliva-toothpaste mixture also has conductivity of similar order of magnitude), with d = 145 µm, and w = 500 µm. L1 and L2 represent domain boundaries. This 2D simulation setup was used to assess various electrical signals in exemplary embodiments.

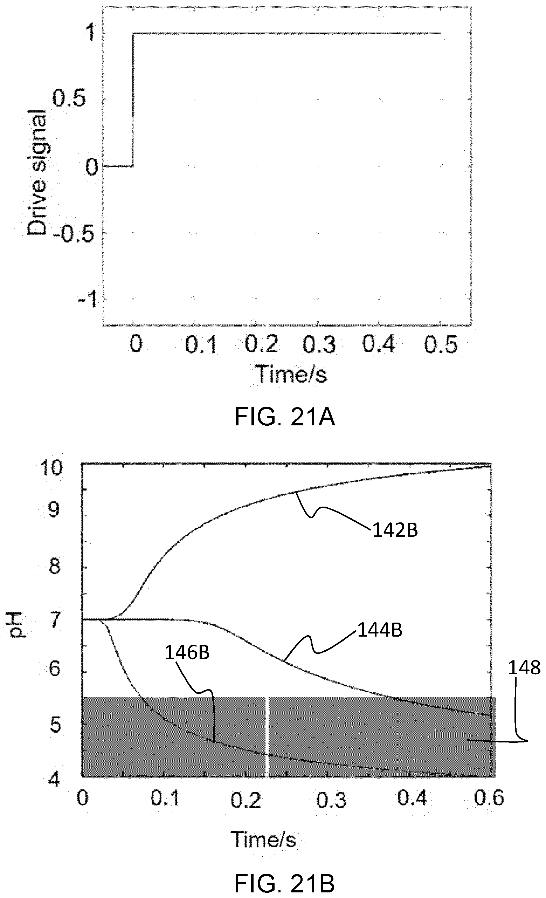

FIG. 21A provides a graph of a non-varying voltage (step signal) across the pair of electrodes 6A, 6B provided by a direct current source, and FIG. 21B provides graphs 142B, 144B, 146B of pH vs time at respective points 142A, 144A, 146A proximal to the electrodes 6A, 6B shown in FIG. 20 when the electrical signal shown in FIG. 21A is provided to the electrodes 6A, 6B.

Whilst the application of a direct current signal leads to a relatively large change and strong drift in pH, a relatively rapid/uncontrolled decrease towards a pH less than pH 5.5 (see region 148 in FIG. 21B) can be observed in graph 146B (corresponding to point 146A in FIG. 20).

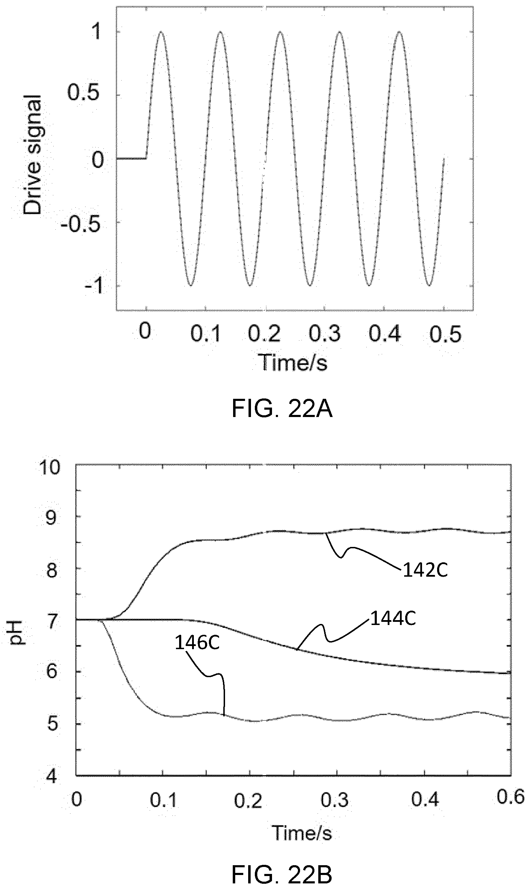

FIG. 22A provides a graph of an electrical signal according to a first example in which the electrical signal is periodic and bipolar, and FIG. 22B provides graphs 142C, 144C, 146C of pH vs time at respective points 142A, 144A, 146A proximal to the electrodes 6A, 6B shown in FIG. 20 when the electrical signal shown in FIG. 22A is provided to the electrodes 6A, 6B.

To enable comparison with FIGs. 22A and 22B, it is noted that the intensity of the non-varying voltage shown in FIG. 21A is normalized.

The electrical signal shown in FIGs. 22A and 22B can be seen to induce relatively rapid stabilization of pH and less overall change of pH, having stabilized around pH 5. Thus, the varying of the electrical signal can enhance the control that can be exerted over the generation of hydrogen ions, and thus the local pH to which the subject's teeth are exposed.

In some embodiments, such as that shown in FIGs. 22A and 22B, the electrical signal comprises a periodic variation in amplitude. This may assist to control the build-up of hydrogen ions, and thus assist in controlling the local pH.

Alternatively or additionally, the electrical signal comprises, or is, a bidirectional waveform. An example of this is shown in FIG. 22A. Alternating the polarity of the electrodes 6A, 6B via such a bidirectional waveform may assist to limit the progression of a low pH "front" emanating from the electrodes 6A, 6B, and stabilize the pH. This is because, following a change in polarity, previously generated hydrogen ions at one of the electrodes 6A, 6B may be neutralized by currently generated hydroxide ions at the same electrode 6A, 6B.

In some embodiments, such as that shown in FIGs. 22A and 22B, the electrical signal comprises, or is, a continuous waveform. In particular, FIG. 22A shows a continuous waveform in the form of a sine wave.

In alternative embodiments, the electrical signal comprises, or is, a pulsed electrical signal. Such a pulsed electrical signal can, for instance, be generated in the manner described above in relation to FIGs. 15A and 15B.

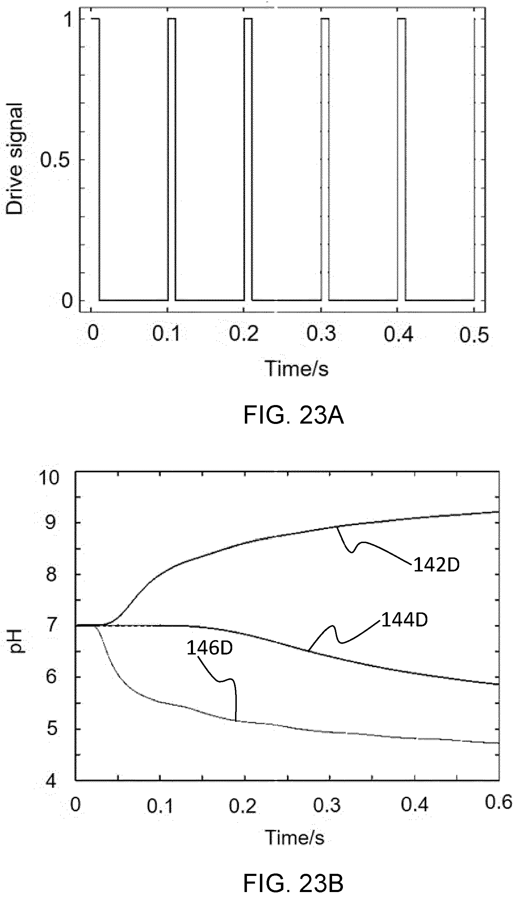

FIG. 23A provides a graph of an electrical signal according to a second example in which the electrical signal is periodic, pulsed (10 Hz, 10% duty cycle) and unipolar, and FIG. 23B provides graphs 142D, 144D, 146D of pH vs time at respective points 142A, 144A, 146A proximal to the electrodes 6A, 6B shown in FIG. 20 when the electrical signal shown in FIG. 23A is provided to the electrodes 6A, 6B.

In contrast to the non-varying voltage of FIGs. 21A and 21B in which the pH reaches an unsafe value of pH 4 relatively rapidly, the pulsed signal of FIGs. 23A and 23B results in a much more gentle and controlled change of pH reaching pH 4.8 after about 0.5 seconds.

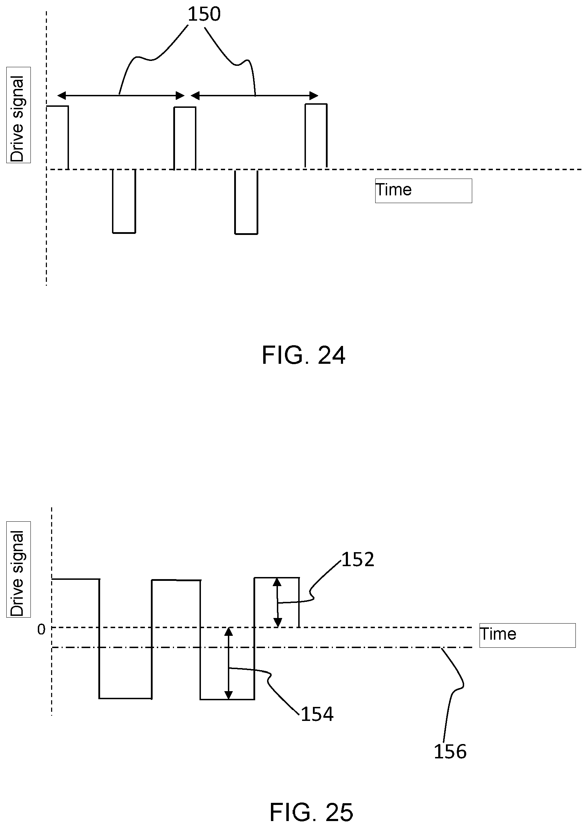

In some embodiments, and referring to FIG. 24, a duty cycle of the pulsed electrical signal is less than or equal to 90%, preferably less than or equal to 75%, most preferably less than or equal to 50%. The duty cycle is the percentage of the ratio of pulse duration, or pulse width, to the total period 150 of the waveform. The associated smaller total current "on" time associated with the pulsed electrical signal relative to the non-varying voltage of FIGs. 21A and 21B can assist to limit the progression of the low pH "front".

In some embodiments, such as that shown in FIG. 24, the electrical (current or voltage) signal is both pulsed and bipolar. The signal in each half cycle can be applied with a given duty cycle. The duty cycle is 50% in the non-limiting example shown in FIG. 24.

A duty cycle associated with a positive component of the bipolar electrical signal may be the same as or different to a duty cycle associated with a negative component of the bipolar electrical signal.

It is noted that in some embodiments, no duty cycle may be employed.

The electrical signal may, in some embodiments, be defined by asymmetric pulsing. Such an asymmetric electrical signal may assist to induce preferential pH change at a particular location.

In some embodiments, such as that shown in FIG. 25, the electrical signal is a bidirectional waveform, and a positive peak amplitude 152 of the bidirectional waveform is different from a negative peak amplitude 154 of the bidirectional waveform. This may result in a continuous, but controlled increase or decrease in pH at a particular location. Thus, the greater control provided by pulsing of the electrical signal relative to the application of a non-varying voltage may be combined with the limiting of creation of "extreme" pH zones near the electrode(s) 6A, 6B due to periodic neutralization provided by the changing polarity.

In some embodiments, one of the positive and negative peak amplitudes 152, 154 is at least 1.1 times the other of the positive and negative peak amplitudes 154, 152.

In the non-limiting example shown in FIG. 25, the negative peak amplitude 154 is 1.5 times the positive peak amplitude 152. The mean signal is represented in FIG. 25 by the dotted line 156.

In some embodiments, the control arrangement 102 is configured to provide, following the electrochemical system 3 being controlled by the processor(s) 4 to provide the electrochemical treatment, the electrical signal to the pair of electrodes 6A, 6B for a predetermined time period before terminating the provision of the electrical signal to the pair of electrodes 6A, 6B. Such a predetermined time period, e.g. in combination with the variation of the electrical signal, may assist to control build-up of hydrogen ions generated by the electrodes 6A, 6B.

This predetermined time period may be the same as or different from a (further) predetermined time period during which the actuator 9 moves in accordance with the electrical signal. For example, the further predetermined period during which the actuator 9 moves in accordance with the electrical signal may be longer than the predetermined period during which the electrical signal is provided to the pair of electrodes 6A, 6B.

FIG. 26 provides a flowchart of a method 200. The method 200 is of controlling an electrochemical system (and optionally an actuator and electrochemical system) of an oral care system further comprising a detection system for detecting dental tartar within a subject's oral cavity. The oral care system may be that of any of the embodiments described herein.

The method 200 comprises obtaining 202, via the detection system, a dental tartar indication indicative of dental tartar within the subject's oral cavity, and controlling 204, based on the dental tartar indication, the electrochemical system to generate ions.

The ions, such as hydrogen ions, zinc ions, tin ions, copper ions, and/or silver ions, may be deliverable into the subject's oral cavity, as previously described. In some embodiments, the method 200 does not include delivery of the ions within the subject's oral cavity, and correspondingly the method 200 does not include any therapeutic treatment step.

FIG. 27 provides a flowchart of a method 300, e.g. a computer-implemented method in the form of a software algorithm, according to a non-limiting illustrative example.

The processor(s) 4 may execute the software method 300 shown in FIG. 27 to establish safe and efficient use of the oral care system 1, and in particular safe and efficient use of an electrochemical tartar treatment mode of the oral care system 1.

In step 302A, the software determines whether tartar is present.

For a spectral measurement, by detecting a local maximum in absorbance (local minimum for reflectance) in the highlighted wavelength windows (see FIGs. 7 and 8), a peak can be found that distinguishes enamel from dental tartar. Spurious results from the noisy spectral data can, for example, be filtered, by setting a high enough "peak prominence" as a criterion for peak detection.

Using a spectral camera 7E, by combining the images from different wavelength bins, enamel can be distinguished from tartar. A simple example of this could be as follows. The reflected image in the 3500-3700 cm⁻¹ region could be normalized (element-wise division of the image matrices) with an image from a 4000-4300 cm⁻¹ wavelength window. The dark spots in this resultant image would then be dental tartar on the bright enamel.

In addition to classical spectral analysis, artificial intelligence (AI)/machine learning models can also be deployed to classify regions as enamel or tartar based on IR spectral features. The models may be trained using ground truth training data generated by the optical spectrum module or using known reference spectra from the above-mentioned cloud database 4M.

A significant advantage of AI models may be that the software method can be made (more) robust against noise due to presence or IR absorption by saliva+toothpaste slurry. The inventors have separately demonstrated that it is possible to segment toothpaste-covered tooth areas from clean areas. A similar technique can be used to identify tartar or enamel during brushing with toothpaste.

Block 302B is a decision point (yes/no) based on the outcome of the analysis of step 302A. If tartar is not detected (N), the software repeats step 302A, and the oral care system 1 continues operating in an initial mechanical and/or fluidic cleaning mode 306.

If based on the analysis tartar is detected (Y), a tartar removal mode is activated and a tartar treatment counter is initiated (i=1) in step 304A. Each count of tartar treatment could be a treatment cycle such as an electric pulse, but may also be related to an overall time criterion, e.g. one treatment = "t" number of seconds of contact of a positive, acid-producing, electrode. Furthermore, a predetermined number of electrochemical treatments X may be defined.

In step 304A, an electrochemical tartar treatment with or without mechanical and/or fluidic cleaning may be implemented. The software may control the electrochemical treatment mode based on predefined settings (e.g. voltage, pulse time etc.). The electrochemical treatment can occur without (=tartar removal mode) or preferably in the presence of mechanical and/or fluidic cleaning (=co-treatment mode). In the co-treatment mode, the processor(s) 4 may be configured to control both the actuator 9 and the electrochemical system 3 based on the tartar indication, as previously described.

In step 302C, analysis of spectra or spectral images during the tartar or co-treatment mode is implemented. In step 302C, acquired spectra or spectral images are analysed to determine how much tartar could be removed during the treatment or whether already sound enamel has appeared at the previously tartar-covered location. Analysis of spectra has already been described in relation to step 302A.

Block 302D is a decision point (yes/no) based on analysis of step 302C. If enamel is detected (e.g. tartar fully removed; Y), the software stops/disables the electrochemical tartar removal mode (Step 304C). If enamel is not detected (N), i.e., tartar is still present based on the analysis, the software remains in the tartar removal or co-treatment mode in which the number of tartar treatments is compared with a predetermined threshold (Step 304B).

Block 304B is a decision point (yes/no) based on comparison of actual treatment number i with predetermined number of electrochemical treatments X.

If the 'No' path (N) is taken at 304B, if the actual treatment number i is less than the predetermined number X, the software further executes steps 304A to 302D (counter i = i+1) and controls the tartar/co-treatment mode. In the specific case that the user moves the treatment device after completing treatments, but before completing the predetermined X number of treatments (to a different tooth, or different location on same tooth), the software may store the treatment number along with the location of the treated area (meaning of location in this case, could be a quadrant of a mouth map as shown in an app, or could also be specific to a tooth on such a quadrant). On the second pass over such a location, the treatment may begin again (Step 304A) starting from a counter value i = A̅ +1.

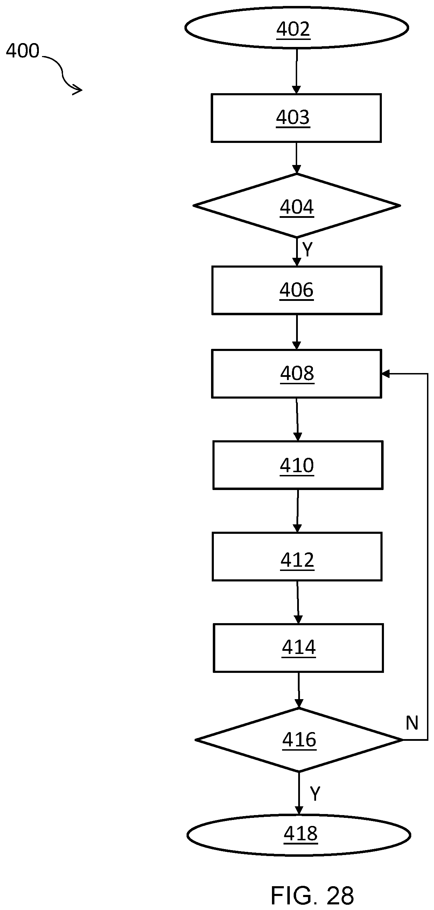

If the 'Yes' path (Y) is taken at 304B, if the actual treatment number i is equal to X (i=X), this indicates that tartar cannot be efficiently removed. Therefore, the processor(s) 4 may control the output device 10 to provide a notification. For example, the user obtains feedback or teledentistry actions are triggered (Step 308). Action triggering can also include directly sending an alarm to a dental practitioner and scheduling an appointment or initiating remote diagnosis (e.g. by sending images of the relevant tooth location to the dental practitioner). Alternatively, also the electrical power of the electrochemical system can be adjusted as further elaborated in Fig. 28.

FIG. 28 provides a flowchart of a method 400 according to yet another example in which an optimal number of electrochemical treatments cycles required for a tooth is determined, along with dynamic adaption of electrochemical power used in different cycles of treatment to minimize possible damage to dental enamel.

The method 400 starts at 402, and an initial spectral signal is analyzed in step 403. At block 404, it is decided whether or not a characteristic tartar spectral pattern is detected. If yes, the logic proceeds to step 406 in which a number of treatment cycles is calculated. The spectral properties of the initial signal can be used to calculate a total number of cycles required for treatment events. This can be based on earlier comparable tartar removal events from the same user, and/or comparable tartar removal events from other users (e.g. from a historical database). In step 408, the electrochemical treatment is provided.

In step 410, a second spectral signal is obtained, and in step 412 a pattern of the second spectral signal is compared with an enamel spectral pattern. In step 414, a treatment power may be reduced based on overlap, e.g. an extent of overlap, of the second spectral signal with the enamel spectral pattern.

At block 416 it is decided whether or not the second spectral signal is within the enamel spectral pattern. If yes, the treatment is stopped at step 418. If not, the logic reverts to step 408.

As previously explained, enamel and tartar may have characteristic spectral patterns. With initiation of electrochemical tartar removal therapy, layers of tartar may be removed from the tooth's surface during each cycle, reducing its thickness and eventually exposing enamel to electrochemical therapy. It may be important to minimize any adverse effect of these methods on enamel. As the tartar layer's thickness diminishes, the spectral pattern may slowly shift from a tartar to an enamel pattern. This spectral information can thus be used to dynamically adapt power/dose of delivered therapy.

In particular, the power/dose of therapy may be reduced as the tartar layer's thickness decreases to protect enamel from any adverse impact. This can be expressed as: Power of treatment (PT) = f(tartar thickness) or Power of treatment (PT) = f(closeness to enamel).

In the above equation, the closeness to enamel can be determined by spectral overlap between the second spectral signal and a typical enamel spectral pattern, e.g. published in literature. Higher overlap may indicate greater closeness to enamel, and a proportional reduction in power may be delivered.

Should the dental tartar indication, e.g. determined via the spectral overlap, be indicative of the dental tartar layer not exhibiting a diminished thickness, or a rate of decrease of the thickness being equal to or below a threshold, during the electrochemical procedure, the processor(s) 4 may be configured to increase the power level, e.g. DC voltage or current, or impedance, used by the electrochemical system 3 to generate the ions and/or may issue the notification to the user, e.g. to the subject's dental care provider.

Thus, the oral care system 1 may provide an electrochemical procedure that is adaptive to relatively stubborn dental tartar deposits.

The above-described processor(s) 4, control arrangement 102 (and/or the drive train circuitry 116) can be implemented in numerous ways, with software and/or hardware, to perform the various required functions. One or more of these components may employ microprocessor(s) that can be programmed using software (e.g., microcode) to perform the functions.

The control arrangement 102/drive train circuitry 116 may, however, be implemented with or without employing a microprocessor, and also may be implemented as a combination of dedicated hardware to perform some functions and a processor, e.g. one or more programmed microprocessors and associated circuitry, to perform other functions.

Examples of controller components that may be employed in various embodiments of the present disclosure include, but are not limited to, conventional microprocessors, application specific integrated circuits (ASICs), and field-programmable gate arrays (FPGAs).

In some examples, the processor(s) 4, control arrangement 102 and/or drive train circuitry 116 is or are associated with one or more storage media such as volatile and non-volatile computer memory such as RAM, PROM, EPROM, and EEPROM. The storage media can be encoded with one or more programs that, when executed on one or more processors and/or controllers, perform the required functions. Various storage media may be fixed within the processor(s) 4, control arrangement 102 and/or drive train circuitry 116 or may be transportable, such that the one or more programs stored thereon can be loaded into the processor(s) 4, control arrangement 102 and/or drive train circuitry 116.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

## Claims

1. An oral care system (1) comprising:
a detection system (2) for detecting dental tartar within a subject's oral cavity;
an electrochemical system (3) for generating ions for delivery within the subject's oral cavity during an electrochemical procedure; and
one or more processors (4) configured to:
obtain, via the detection system, a dental tartar indication indicative of dental tartar within the subject's oral cavity; and
control the electrochemical procedure provided by the electrochemical system based on the dental tartar indication.

2. The oral care system (1) according to claim 1, wherein the detection system (2) comprises an optical detection system for optically detecting dental tartar within the subject's oral cavity; optionally wherein the optical detection system comprises an imaging system.

3. The oral care system (1) according to claim 1 or claim 2, wherein the detection system comprises a spectral detection system configured to spectroscopically detect the presence of dental tartar within the subject's oral cavity.

4. The oral care system (1) according to claim 3, wherein the one or more processors (4) is or are configured to execute a spectral comparison algorithm to distinguish between dental tartar and enamel, the dental tartar indication comprising a result of the spectral comparison algorithm.

5. The oral care system (1) according to claim 3 or claim 4, wherein the spectroscopic detection of the presence of dental tartar within the subject's oral cavity comprises infrared spectroscopic detection.

6. The oral care system (1) according to any one of claims 1 to 5, wherein the electrochemical system (3) comprises a control arrangement (102), and one or more pairs of electrodes (6A, 6B; 6C, 6D) for contacting an aqueous solution, wherein the one or more processors (4) is or are configured to, based on the dental tartar indication indicating the presence of dental tartar, trigger the control arrangement to provide an electrical signal that causes at least one electrode of the one or more pairs of electrodes to generate hydrogen ions from water in the aqueous solution to deliver within the subject's oral cavity.

7. The oral care system (1) according to claim 6, wherein said at least one electrode of the one or more pairs of electrodes (6A, 6B; 6C, 6D) is insertable into the subject's oral cavity, said aqueous solution comprising the subject's saliva and/or one or more oral care agents.

8. The oral care system (1) according to claim 6 or claim 7, wherein a variation of the electrical signal limits a rate of change of generation of the hydrogen ions and/or promotes hydrogen ion neutralization.

9. The oral care system (1) according to any one of claims 1 to 8, comprising at least one cleaning element (8) for mechanically and/or fluidically cleaning inside the subject's oral cavity; optionally wherein the at least one cleaning element comprises bristles and/or a fluid-delivering nozzle for cleaning surfaces inside the subject's oral cavity.

10. The oral care system (1) according to claim 9, wherein the one or more processors (4) is or are configured to control movement of and/or fluid delivery from the at least one cleaning element (8).

11. The oral care system (1) according to any one of claims 1 to 10, wherein the one or more processors (4) is or are configured to control the electrochemical system (3) to:
initiate generation of the ions based on the dental tartar indication being indicative of dental tartar being present at a location within the subject's oral cavity, and to terminate generation of the ions based on detection of enamel at the location; and/or
adjust a power used by the electrochemical system to generate the ions based on the dental tartar indication being indicative of an amount of dental tartar present at a location within the subject's oral cavity.

12. The oral care system (1) according to any one of claims 1 to 11, comprising an oral care assembly (5) that is insertable into the subject's oral cavity, the electrochemical system (3) comprising at least one ion generation electrode (6A, 6B; 6C, 6D) for generating said ions, and the detection system (2) comprising at least one detection element (7A, 7B, 7C, 7D; 7E), wherein the at least one ion generation electrode and the at least one detection element are included in the oral care assembly.

13. The oral care system (1) according to any one of claims 1 to 12, comprising an output device (10), wherein the one or more processors (4) is or are configured to control the output device, based on the dental tartar indication, to provide a notification to a user; optionally wherein the user is at least one of the subject and the subject's dental care provider.

## Patentansprüche

1. Mundpflegesystem (1), umfassend:
ein Detektionssystem (2) zum Detektieren von Zahnstein in der Mundhöhle einer Person;
ein elektrochemisches System (3) zum Erzeugen von Ionen zur Abgabe in der Mundhöhle der Person während eines elektrochemischen Vorgangs; und
einen oder mehrere Prozessoren (4), die so konfiguriert sind, dass sie:
über das Detektionssystem einen Zahnsteinhinweis erhalten, der auf Zahnstein in der Mundhöhle der Person hinweist; und
den elektrochemischen Vorgang, der über das elektrochemische System bereitgestellt wird, auf Basis des Zahnsteinhinweises steuern.

2. Mundpflegesystem (1) nach Anspruch 1, wobei das Detektionssystem (2) ein optisches Detektionssystem zum optischen Detektieren von Zahnstein in der Mundhöhle der Person umfasst; gegebenenfalls wobei das optische Detektionssystem ein Bildgebungssystem umfasst.

3. Mundpflegesystem (1) nach Anspruch 1 oder Anspruch 2, wobei das Detektionssystem ein spektrales Detektionssystem umfasst, das so konfiguriert ist, dass es das Vorhandensein von Zahnstein in der Mundhöhle der Person spektroskopisch detektiert.

4. Mundpflegesystem (1) nach Anspruch 3, wobei der eine oder die mehreren Prozessoren (4) so konfiguriert ist bzw. sind, einen Spektralvergleichsalgorithmus auszuführen, um zwischen Zahnstein und Zahnschmelz zu unterscheiden, wobei der Zahnsteinhinweis ein Ergebnis des Spektralvergleichsalgorithmus umfasst.

5. Mundpflegesystem (1) nach Anspruch 3 oder Anspruch 4, wobei die spektroskopische Detektion des Vorhandenseins von Zahnstein in der Mundhöhle der Person infrarotspektroskopische Detektion umfasst.

6. Mundpflegesystem (1) nach einem der Ansprüche 1 bis 5, wobei das elektrochemische System (3) eine Steuerungsanordnung (102) und ein oder mehrere Elektrodenpaare (6A, 6B; 6C, 6D) zum Inkontaktbringen mit einer wässrigen Lösung umfasst, wobei der eine oder die mehreren Prozessoren (4) so konfiguriert ist bzw. sind, auf Basis des Zahnsteinhinweises, der auf das Vorhandensein von Zahnstein hinweist, die Steuerungsanordnung auszulösen, um ein elektrisches Signal bereitzustellen, das bewirkt, dass mindestens eine Elektrode des einen oder der mehreren Elektrodenpaare Wasserstoffionen aus Wasser in der wässrigen Lösung erzeugt, die in der Mundhöhle der Person abgegeben werden sollen.

7. Mundpflegesystem (1) nach Anspruch 6, wobei die mindestens eine Elektrode des einen oder der mehreren Elektrodenpaare (6A, 6B; 6C, 6D) in die Mundhöhle der Person eingeführt werden kann, wobei die wässrige Lösung den Speichel der Person und/oder ein oder mehrere Mundpflegemittel umfasst.

8. Mundpflegesystem (1) nach Anspruch 6 oder Anspruch 7, wobei eine Variation des elektrischen Signals eine Änderungsrate der Erzeugung der Wasserstoffionen begrenzt und/oder die Neutralisierung von Wasserstoffionen fördert.

9. Mundpflegesystem (1) nach einem der Ansprüche 1 bis 8, das mindestens ein Reinigungselement (8) zum mechanischen und/oder fluidischen Reinigen innerhalb der Mundhöhle der Person umfasst;
gegebenenfalls wobei das mindestens eine Reinigungselement Borsten und/oder eine Fluidabgabedüse zum Reinigen von Oberflächen innerhalb der Mundhöhle der Person umfasst.

10. Mundpflegesystem (1) nach Anspruch 9, wobei der eine oder die mehreren Prozessoren (4) so konfiguriert ist bzw. sind, Bewegung von und/oder Fluidabgabe aus dem mindestens einen Reinigungselement (8) zu steuern.

11. Mundpflegesystem (1) nach einem der Ansprüche 1 bis 10, wobei der eine oder die mehreren Prozessoren (4) so konfiguriert ist bzw. sind, das elektrochemische System (3) so zu steuern, dass es:
Erzeugung der Ionen auf Basis dessen einleitet, dass der Zahnsteinhinweis auf das Vorhandensein von Zahnstein an einer Stelle in der Mundhöhle der Person hinweist, und Erzeugung der Ionen auf Basis dessen beendet, dass an der Stelle Zahnschmelz detektiert wird; und/oder
eine Leistung, die vom elektrochemischen System verwendet wird, um die Ionen zu erzeugen, auf Basis dessen anpasst, dass der Zahnsteinhinweis auf eine Menge an Zahnstein hinweist, die an einer Stelle in der Mundhöhle der Person vorhanden ist.

12. Mundpflegesystem (1) nach einem der Ansprüche 1 bis 11, das eine Mundpflegebaugruppe (5), die in die Mundhöhle der Person eingeführt werden kann, das elektrochemische System (3), das mindestens eine lonenerzeugungselektrode (6A, 6B; 6C, 6D) zum Erzeugen der Ionen umfasst, und das Detektionssystem (2) umfasst, das mindestens ein Detektionselement (7A, 7B, 7C, 7D; 7E) umfasst, wobei die mindestens eine lonenerzeugungselektrode und das mindestens eine Detektionselement in der Mundpflegebaugruppe beinhaltet sind.

13. Mundpflegesystem (1) nach einem der Ansprüche 1 bis 12, das eine Ausgabevorrichtung (10) umfasst, wobei der eine oder die mehreren Prozessoren (4) so konfiguriert ist bzw. sind, die Ausgabevorrichtung auf Basis des Zahnsteinhinweises zu steuern, einem Benutzer eine Meldung bereitzustellen; gegebenenfalls wobei der Benutzer mindestens eines von der Person oder dem zahnmedizinischen Versorger der Person ist.

## Revendications

1. Système d'hygiène buccale (1) comprenant :
un système de détection (2) pour détecter du tartre dentaire dans la cavité buccale d'un sujet ;
un système électrochimique (3) pour générer des ions destinés à être délivrés dans la cavité buccale du sujet au cours d'une procédure électrochimique ; et
un ou plusieurs processeurs (4) configurés pour :
obtenir, par le biais du système de détection, une indication de tartre dentaire indiquant du tartre dentaire dans la cavité buccale du sujet ; et
commander la procédure électrochimique fournie par le système électrochimique sur la base de l'indication de tartre dentaire.

2. Système d'hygiène buccale (1) selon la revendication 1, dans lequel le système de détection (2) comprend un système de détection optique pour détecter optiquement du tartre dentaire dans la cavité buccale du sujet ; en option, dans lequel le système de détection optique comprend un système d'imagerie.

3. Système d'hygiène buccale (1) selon la revendication 1 ou la revendication 2, dans lequel le système de détection comprend un système de détection spectrale configuré pour détecter par spectroscopie la présence de tartre dentaire dans la cavité buccale du sujet.

4. Système d'hygiène buccale (1) selon la revendication 3, dans lequel le ou les processeurs (4) sont configurés pour exécuter un algorithme de comparaison spectrale afin de distinguer le tartre dentaire de l'émail, l'indication de tartre dentaire comprenant un résultat de l'algorithme de comparaison spectrale.

5. Système d'hygiène buccale (1) selon la revendication 3 ou la revendication 4, dans lequel la détection par spectroscopie de la présence de tartre dentaire dans la cavité buccale du sujet comprend une détection par spectroscopie infrarouge.

6. Système d'hygiène buccale (1) selon l'une quelconque des revendications 1 à 5, dans lequel le système électrochimique (3) comprend un dispositif de commande (102) et une ou plusieurs paires d'électrodes (6A, 6B ; 6C, 6D) destinées à entrer en contact avec une solution aqueuse, dans lequel le ou les processeurs (4) sont configurés pour, sur la base de l'indication de tartre dentaire indiquant la présence de tartre dentaire, déclencher le dispositif de commande afin de fournir un signal électrique qui amène au moins une électrode de la ou des paires d'électrodes à générer des ions d'hydrogène à partir de l'eau dans la solution aqueuse pour les délivrer dans la cavité buccale du sujet.

7. Système d'hygiène buccale (1) selon la revendication 6, dans lequel au moins une électrode parmi une ou plusieurs paires d'électrodes (6A, 6B ; 6C, 6D) est insérable dans la cavité buccale du sujet, la solution aqueuse comprenant la salive du sujet et/ou un ou plusieurs produits d'hygiène buccale.

8. Système d'hygiène buccale (1) selon la revendication 6 ou la revendication 7, dans lequel une variation du signal électrique limite un taux de variation de la génération des ions d'hydrogène et/ou favorise la neutralisation des ions d'hydrogène.

9. Système d'hygiène buccale (1) selon l'une quelconque des revendications 1 à 8, comprenant au moins un élément de nettoyage (8) pour le nettoyage mécanique et/ou fluidique de l'intérieur de la cavité buccale du sujet ;
optionnellement dans lequel l'au moins un élément de nettoyage comprend des poils et/ou une buse de distribution de fluide pour nettoyer les surfaces à l'intérieur de la cavité buccale du sujet.

10. Système d'hygiène buccale (1) selon la revendication 9, dans lequel le ou les processeurs (4) sont configurés pour commander le mouvement et/ou la distribution de fluide à partir d'au moins un élément de nettoyage (8).

11. Système d'hygiène buccale (1) selon l'une quelconque des revendications 1 à 10, dans lequel le ou les processeurs (4) sont configurés pour commander le système électrochimique (3) afin de :
initier la génération des ions sur la base de l'indication de tartre dentaire indiquant la présence de tartre dentaire à un endroit de la cavité buccale du sujet, et mettre fin à la génération des ions sur la base de la détection d'émail à cet endroit ; et/ou
ajuster une puissance utilisée par le système électrochimique pour générer les ions en fonction de l'indication de tartre dentaire, celle-ci indiquant une quantité de tartre dentaire présent à un endroit donné dans la cavité buccale du sujet.

12. Système d'hygiène buccale (1) selon l'une quelconque des revendications 1 à 11, comprenant un ensemble d'hygiène buccale (5) qui peut être inséré dans la cavité buccale du sujet, le système électrochimique (3) comprenant au moins une électrode de génération d'ions (6A, 6B ; 6C, 6D) pour générer lesdits ions, et le système de détection (2) comprenant au moins un élément de détection (7A, 7B, 7C, 7D ; 7E), dans lequel l'au moins une électrode de génération d'ions et l'au moins un élément de détection sont inclus dans l'ensemble d'hygiène buccale.

13. Système d'hygiène buccale (1) selon l'une quelconque des revendications 1 à 12, comprenant un dispositif de sortie (10), dans lequel le ou les processeurs (4) sont configurés pour commander le dispositif de sortie, sur la base de l'indication de tartre dentaire, afin de fournir une notification à un utilisateur ; optionnellement dans lequel l'utilisateur est au moins le sujet et le fournisseur de soins dentaires du sujet.
